(19) Europäisches Patentamt
European Patent Office
Office européen des brevets

(11) Publication number: **0 214 303 A1**

(12) # EUROPEAN PATENT APPLICATION

published in accordance with Art. 158(3) EPC

(21) Application number: 86901502.4

(22) Date of filing: 21.02.86

Data of the international application taken as a basis:

(86) International application number: PCT/JP86/00085

(87) International publication number: WO86/04898 (28.08.86 86/19)

(51) Int.Cl.[4]: C 07 D 487/14, A 61 K 31/40

(30) Priority: 22.02.85 JP 33880/85
13.03.85 JP 50095/85
24.09.85 JP 210442/85

(43) Date of publication of application: 18.03.87 Bulletin 87/12

(84) Designated Contracting States: DE FR GB IT

(71) Applicant: KYOWA HAKKO KOGYO KABUSHIKI KAISHA
6-1, Ohte-machi 1-chome
Chiyoda-ku Tokyo 100(JP)

(72) Inventor: SHIRAHATA, Kunikatsu
4-11-5, Iwadominami
Komae-shi Tokyo 201(JP)

(72) Inventor: KASAI, Masaji
3-12-15, Kugenumamatsugaoka
Fujisawa-shi Kanagawa 251(JP)

(72) Inventor: KONO, Motomichi
1132-8, Kiso-machi
Machida-shi Tokyo 194(JP)

(72) Inventor: MORIMOTO, Makoto
1188, Shimotogari
Nagaizumi-cho, Sunto-gun Shizuoka 411(JP)

(72) Inventor: ASHIZAWA, Tadashi
3236-13, Ooka
Numazu-shi Shizuoka 410(JP)

(72) Inventor: SAITO, Yutaka
Dainikenyuryo 4-17-9
Morino, Machida-shi Tokyo 194(JP)

(74) Representative: Casalonga, Axel et al,
BUREAU D.A. CASALONGA - JOSSE Morassistrasse 8
D-8000 Munich 5(DE)

(54) **DISULFIDE COMPOUNDS AND ANTINEOPLASTIC AGENTS.**

(57) Mitomycin derivatives represented by general formula (I), wherein X represents $X_1$ or $X_2$, $X_1$ represents an alkyl group, a cycloalkyl group (optionally substituted), a pyridyl group, an unsubstituted or substituted phenyl group, a residue of an amino acid having a thiol group or a dipeptide or tripeptide residue containing the amino acid excluding the thiol group, $X_2$ represents a group represented by formula (II), (wherein $A_1$ represents $-(CH_2)_m-$ (wherein m represents an integer of 3 to 12) or $-(CH_2CH_2O)_n-CH_2CH_2-$ (wherein n represents an integer of 1 to 5), Y, Z, $R_1$, and $R_2$ are the same as defined with (I), provided that A represents $-(CH_2)_\ell-$ (wherein $\ell$ represents an integer of 3 to 8) when X is $X_1$ and A represents $A_1$ when X is $X_2$, Y and Z each represents H or methyl, and one of $R_1$ and $R_2$ represents a carbamoyloxymethyl group and the other represents H when X is $X_1$, and one of them represents a carbamoyloxymethyl group and the other represents H, or both are bound to each other to form an exo-methylene group (= $CH_2$) when X is $X_2$. These compounds are superior to known mitomycin derivatives having two methylene units between a nitrogen atom at 7-position and a SS grouping in at least one of CI value (chemotherapeutic index), reduction of side effects, and solubility in water.

EP 0 214 303 A1

TITLE MODIFIED
see front page



SPECIFICATION

# DISULFIDE COMPOUND AND ANTI-TUMOR AGENT

## Technical Field

This invention relates to a novel mitomycin derivative having an antibacterial activity and an anti-tumor activity and having dithio group (-S-S-) at the 7-position, an anti-tumor agent containing the same, and bis(ω-aminooxaalkyl)disulfides useful as starting materials for synthesis of the mitomycin derivatives, derivatives thereof and acid adduct salts thereof.

## Background Art

Mitomycins are generally known as antibiotics having an antibacterial activity and anti-tumor activity. Typical mitomycins are mitomycin A, mitomycin B, mitomycin C and porfiromycin (the foregoing compounds are disclosed in Merck Index, 10th edition); mitomycin D and mitomycin E (the foregoing compounds are disclosed in Japanese Published Unexamined Patent Application No. 122797/1979); mitomycin F and mitomycin J (the foregoing compounds are disclosed in Japanese Published Unexamined Patent Application No. 45322/1980); mitomycin G and mitomycin H (the foregoing compounds are disclosed in Japanese Published Unexamined Patent Application No. 15408/1980), etc. These mitomycins can be obtained by culturing a strain of Streptomyces caespitosus. Recently, the absolute configurations of mitomycins have been corrected [J. Am. Chem. Soc., 105, 7199 (1983)]. The structures of said various mitomycins as corrected accordingly are shown below.

Typical mitomycins obtained from the natural products:

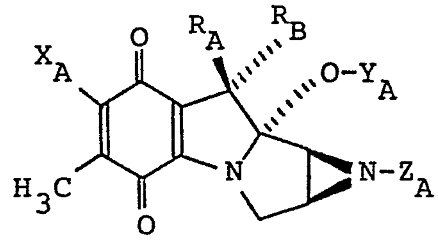

| Mitomycin | $X_A$ | $Y_A$ | $Z_A$ | $R_A$ | $R_B$ |
|---|---|---|---|---|---|
| A | $OCH_3$ | $CH_3$ | H | a | H |
| B | $OCH_3$ | H | $CH_3$ | H | a |
| C | $NH_2$ | $CH_3$ | H | a | H |
| D | $NH_2$ | H | $CH_3$ | H | a |
| E | $NH_2$ | $CH_3$ | $CH_3$ | H | a |
| F | $OCH_3$ | $CH_3$ | $CH_3$ | a | H |
| G | $NH_2$ | $CH_3$ | $CH_3$ | Combined into $=CH_2$ | |
| H | $OCH_3$ | H | $CH_3$ | Combined into $=CH_2$ | |
| J | $OCH_3$ | $CH_3$ | $CH_3$ | H | a |
| Porfiromycin | $NH_2$ | $CH_3$ | $CH_3$ | a | H |

wherein "a" represents - $CH_2OCONH_2$.

Mitomycins have a distinguished anti-tumor activity on one hand, but have side effects such as reduction in leukocytes, etc. on the other hand. Thus, many derivatives have been synthesized in order to intensify the activity or reduce the toxicity.

Among these mitomycin derivatives, the derivatives with a modified amino group at the 7-position include mitomycin C and porfiromycin derivatives with a modified amino group at the 7-position. Examples of the derivatives are disclosed, for example, in US Patent No. 4,268,676, Japanese Published Unexamined Patent Application Nos. 92288/1981 and 188590/1982, J. Med. Chem., 24, 975 - 981 (1981), J. Med. Chem., 26, 16 - 20 (1983), J. Med. Chem., 26, 1453 - 1457 (1983). These literatures also

disclose that the mitomycin derivatives with a modified amino group at the 7-position show an anti-tumor activity in vivo. Among the mitomycin derivatives whose amino group at the 7-position is modified, those pertinent to the present invention are mitomycin derivatives whose substituents at the 7-position are a 2-thiazolamino group, a 2-thienylmethylamino group, and a (4-sulfonamidophenyl) methylamino group (as disclosed in Japanese Published Unexamined Patent Application No. 92288/1981); mitomycin derivatives whose substituents at the 7-position are a 2-mercaptoethylamino group, a 2-ethylthioethylamino group, a thiomorpholino group, a thiazolidyl group, 4-mercapto-anilino group, 2-(4-methylthiazolyl) amino group, 2-(5-methyl-1,3,4-thiadiazolyl) amino group, 4-(2,1,3-benzo-thiadiazolyl) amino group (as disclosed in Japanese Published Unexamined Patent Application No. 188590/1982), and mitomycin derivatives having a group represented by $-NHCH_2CH_2SSCH_2CH_2NH-$ at the 7-position, for example, 7-N,7'-N'-dithiodiethylenedimitomycin C, 7-N,7'-N'-dithiodiethylenedimitomycin D, etc. (EP 0116208A1, Japanese Published Unexamined Patent Application No. 104386/1984). Further, there are mitomycin derivatives whose substituent at the 7-position is represented by $RSS(CH_2)_2NH-$, for example, those whose R is an alkyl group or a substituted alkyl group, e.g. 7-N-propyldithioethylmitomycin C, 7-N-methoxycarbonyl-methyldithioethylmitomycin C, 7-N-[2-(2-hydroxyethyldithio) ethyl] mitomycin D, etc. (EP 0116208A1, Japanese Published Unexamined Patent Application No. 175493/1984), those whose R is an aromatic ring having a substituent, for example, 7-N-[2-(4-acetamidophenyldithio)-ethyl] mitomycin C (EP 0116208A1, Japanese Published Unexamined Patent Application No. 175493/1984), 7-N-[2-(4-fluorophenyldithio)ethyl] mitomycin C (EP 0163550A2, Japanese Published Unexamined Patent Application No. 255789/1985), and those whose R is represented by a residue of an amino acid containing a thiol group, or by a peptide containing the amino acid residue, for

example, 7-N-[2-[(L-cystein-S-yl)thio]ethyl] mitomycin D, 7-N-[2-[glysino-L-cystein-S-yl)thio]ethyl] mitomycin C, etc. (EP 0163550A2, Japanese Published Unexamined Patent Application No. 255789/1985).

Among the mitomycin derivatives having $RSS(CH_2)_2NH$ at the 7-position, mitomycin C derivatives having R-SS-$alk_2$ must be particularly mentioned (Japanese Published Unexamined Patent Application No. 205382/1984), where the disclosed R has overlapping definitions with R of the said Japanese Published Unexamined Patent Application No. 175493/1984. The Japanese Published Unexamined Patent Application No. 205382/1984 further discloses R-SS-$alk_2$-$NH_2$ for reaction with mitomycin having a substituent which can readily be substituted with an amino group, for example, a methoxy group at the 7-position, and further R-SH as a starting material for R-SS-$alk_2$-$NH_2$. However, the said Japanese Published Unexamined Patent Application No. 205382/1984 states that $alk_2$ in R-SS-$alk_2$-$NH_2$ represents a straight or branched alkylene group having 2 to 6 carbon atoms which can have any substituent, but the specific exemplification of $alk_2$ is limited to a dimethylene chain ($-CH_2CH_2-$). In the general description, 6-[(cyclopropyl) methyldithio] hexylamine whose $alk_2$ is hexamethylene is mentioned as the starting material, but there is no disclosure of an example of preparing a desired compound from the said starting material.

Mitomycin derivatives with more distinguished properties are in demand. The present inventors have found that the methylene chain existing between the sulfur atom and the nitrogen atom at the 7-position of mitomycin has a large influence on a biological activity, particularly an anti-tumor activity, and as a result of further detailed study, have obtained compounds having a more distinguished anti-tumor activity, compounds having a reduced side effect, and furthermore mitomycin derivatives having a water solubility which is advantageous for medical preparation.

Further, bis(ω-aminoalkyl) disulfides represented by the formula (A):

$$[-S-(CH_2)_tNH_2]_2 \cdot 2HX \qquad (A)$$

(wherein t is an integer of 1 to 10, and X is a halogen atom) and processes for preparing these compounds have heretofore been known, and can be outlined as follows (literatures are summarized in the following table).

(1) Literatures on the compounds represented by the formula (A):

Compounds where t = 1 : Literature 1
Compounds where t = 2 : Literature 2, etc.
Compounds where t = 3 : Literatures 3, 4, etc.
Compounds where t = 4, 5, and 6 : Literatures 4, 5, etc.
Compounds where t = 8 and 10 : Literature 5, etc.

(2) Processes for preparing compounds (A):

(A) Process comprising reacting sodium thiosulfate with α-haloalkyl-ω-phthalimide, converting the product to bis(ω-phthalimidoalkyl) disulfide through oxidation reaction with iodine, and removing the phthal group therefrom in an organic acid such as acetic acid, propionic acid, etc., or hydrochloric acid or hydrobromic acid, thereby obtaining the compound (A) (Literatures 4, 5, etc.).

(B) Process comprising reacting ω-hydroxyalkylamine with chlorosulfonic acid, thereby obtaining ω-sulfatoalkylamine (Literatures 6, 7, etc.), reacting the amine with carbon disulfide in alkali, thereby obtaining the corresponding cyclic dithiocarbamate (Literature 7), then hydrolyzing the cyclic dithiocarbamate with an acid such as hydrochloric acid, etc., thereby obtaining ω-mercaptoalkylamine (Literature 7), and oxidizing the amine with hydrogen peroxide, etc., thereby synthesizing the compounds (A) (Literature 8).

Table

Examples of literatures disclosing bis(ω-aminoalkyl)disulfides

| Literature No. | Literature |
|---|---|
| 1 | J. Ong. Chem., 38, 916 (1973) |
| 2 | Merk Index. 10th edition 2771 (1983) |
| 3 | Japanese Published Examined Patent Application No. 54314/1981 |
| 4 | J. Pharm. Science. 71, 799 (1982) |
| 5 | Liebigs Ann. Chem., 574, 131 (1951) |
| 6 | J. Am. Chem. Soc., 75, 4101 (1953) |
| 7 | J. Chem. Soc., (C), 1373 (1967) |
| 8 | Japanese Published Unexamined Patent Application No. 75470/1981 |

Compounds (A) are useful substances for starting materials for the synthesis of medicaments, but derivatives synthesized from compounds (A) as starting materials have greatly reduced solubility in water owing to a decrease in the hydrophilic property. Water solubility is a very important factor for some medicaments, and particularly in case of administration in the form of an injecting agent, the water solubility is an essential condition. To use sparingly water-soluble compounds in an injecting agent, solubilizers are often used, but in that case the side effect by them sometimes offers a problem. As a result of studies on the synthesis directed to development of novel mitomycin derivatives under these circumstances, the present inventors have found that bis(ω-aminooxaalkyl) disulfides, etc. can serve as distinguished starting materials for the synthesis of

medicaments for the following two reasons, and have established the present invention:

(1) Derivatives obtained by reaction of the present bis (ω-aminooxaalkyl) disulfides, etc. with mitomycin have medical properties such as antibacterial activity, etc.

(2) Derivatives obtained by reaction of the bis(ω-aminooxaalkyl) disulfides, etc. with mitomycin have a higher water solubility than the derivatives obtained by reaction of the corresponding compound (A) with mitomycin.

Disclosure of Invention

Mitomycin derivatives having a distinguished antibacterial and anti-tumor activity according to the present invention are represented by the following formula (I):

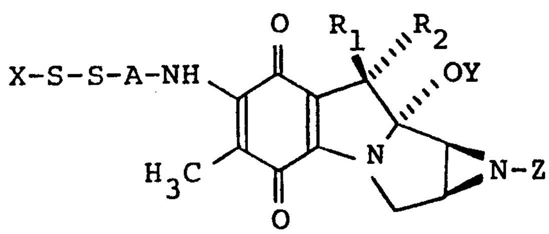

(I)

{wherein, X is $X_1$ or $X_2$, and $X_1$ is an alkyl group having 1 - 7 carbon atoms, a cycloalkyl group having 3 - 7 carbon atoms (the alkyl or cycloalkyl group may be substituted with 1 - 3 of hydroxyl group(s), one amino group or one lower alkanoyl amino group), a pyridyl group, an unsubstituted or substituted phenyl group (the substituent is a lower alkyl group, a hydroxyl group, a lower alkoxy group, a nitro group, an amino group, a lower alkylamino group, a lower alkanoylamino group or a halogen atom, and number of the substituent is an integer of 1 - 5), or a residue of an amino acid having a thiol group or a dipeptide or tripeptide containing a residue of the amino acid and the residue being free from the thiol group, where

the amino acid, the dipeptide and the tripeptide may be those whose carboxyl groups are protected as lower alkyl ester groups and/or whose amino groups are protected by lower alkanoyl groups, or whose carboxyl groups may form an alkali metal salt, an ammonium salt or an organic amine adduct salt; $X_2$ is a group represented by the formula:

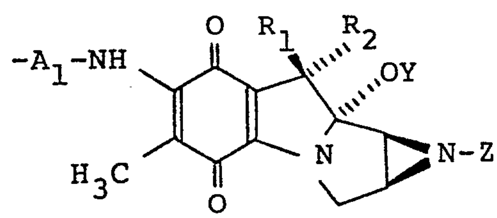

[wherein, $A_1$ is $-(CH_2)_m-$ (wherein, m is an integer of 3 - 12) or $-(CH_2CH_2O)_n-CH_2CH_2-$ (wherein, n is an integer of 1 - 5), and Y, Z, $R_1$ and $R_2$ are as defined in the formula (I)]; when X is $X_1$, A is $-(CH_2)_\ell-$ (wherein, $\ell$ is an integer of 3 - 8) and when X is $X_2$, A is $A_1$; Y and Z are a hydrogen atom or a methyl group; when X is $X_1$, one of $R_1$ and $R_2$ is a carbamoyloxymethyl group, and the other is a hydrogen atom; when X is $X_2$, one of $R_1$ and $R_2$ is a carbamoyloxymethyl group, and the other is hydrogen atom, or $R_1$ and $R_2$ are combined to represent an exo-methylene group ( $=CH_2$ ) } [hereinafter referred to as compound (I). Compounds with other formula numbers are hereinafter likewise referred to].

In the definition of X in the formula (I), the alkyl group having 1 to 7 carbon atoms may be straight or branched, and includes methyl, ethyl, n-propyl, i-propyl, n-butyl, s-butyl, t-butyl, etc. The cycloalkyl group having 3 to 7 carbon atoms includes cyclopentyl, cyclohexyl, etc. The lower alkanoylamino as a substituent on these alkyl and cycloalkyl group has 1 to 3 carbon atoms and includes, for example, acetamido, etc. Examples of the substituted alkyl and substituted cycloalkyl include 2-hydroxyethyl, 3-hydroxypropyl, 2,3-dihydroxypropyl, 2-aminoethyl, 3-aminopropyl, 4-aminobutyl, 2-acetamidoethyl,

4-hydroxycyclohexyl, etc.

Pyridyl is exemplified by 2-pyridyl, 4-pyridyl, etc.

In the definition of the substituent of the substituted phenyl group, the lower alkyl group is straight or branched alkyl groups having 1 to 5 carbon atoms, such as methyl, ethyl, i-propyl, n-butyl, n-pentyl, etc., the lower alkoxy group is alkoxy groups having 1 to 3 carbon atoms, such as methoxy, ethoxy, i-propoxy, etc., the lower alkylamino group is alkylamino groups having 1 to 3 carbon atoms, such as methylamino, ethylamino, etc., the lower alkanoylamino group has 1 to 3 carbon atoms, such as formamido, acetamido, n-propionamido, etc., and the halogen atom includes fluorine, chlorine, bromine, etc. Preferable examples of these substituents are those whose number(s) is/are 1 or 2 and are the same or different lower alkyl groups, hydroxyl groups, lower alkoxy groups, nitro groups, amino groups, lower alkylamino groups or lower alkanoylamino groups, or are halogen atoms whose number(s) is/are 1 - 5. Examples of the unsubstituted or substituted phenyl group include phenyl, 3-aminophenyl, 4-aminophenyl, 3,4-diaminophenyl, 2-acetamidophenyl, 3-acetamidophenyl, 4-acetamidophenyl, 4-acetamido-3-aminophenyl, 3-acetamido-4-aminophenyl, 4-nitrophenyl, 2-methoxyphenyl, 3-methoxyphenyl, 4-methoxyphenyl, 4-ethoxyphenyl, 4-methylphenyl, 4-ethylphenyl, 2-chlorophenyl, 4-chlorophenyl, 2-bromophenyl, 4-bromophenyl, 4-fluorophenyl, 2,5-dichlorophenyl, 2,6-dichlorophenyl, 3,4-dichlorophenyl, 2,4,5-trichlorophenyl, 2,3,5,6-tetrafluorophenyl, 4-hydroxyphenyl, 3,4-dihydroxyphenyl, etc.

In the definition of X, the amino acid having a thiol group or the dipeptide or tripeptide containing the residue of the amino acid includes cysteine, homocysteine, penicillamine, asparaginocysteine, glutaminocysteine, glycinocysteine, phenylalaninocysteine, tyrocinocysteine, glutathione, etc. Steric configuration of these amino acids or amino acid residues may be L, D, or DL.

The carboxyl groups of these amino acids, dipeptides, and tripeptides may be protected as lower alkyl ester groups, and the lower alkyl group includes methyl and ethyl, and the lower alkanoyl group for protecting the amino group is alkanoyl groups having 1 to 3 carbon atoms and includes, for example, formyl, acetyl, etc. The alkali metal capable of forming a salt with the carboxyl group includes sodium, potassium, etc., and the organic amine includes triethylamine, pyridine, etc.

A procedure for preparing the compound (I) will be described below, separating the case where X is $X_1$ from the case where X is $X_2$.

(1) In case of X being $X_1$ [compound (I) in this case is referred to as compound (I-1)]

In the Compound (I-1), those represented by the formula (I-1-1):

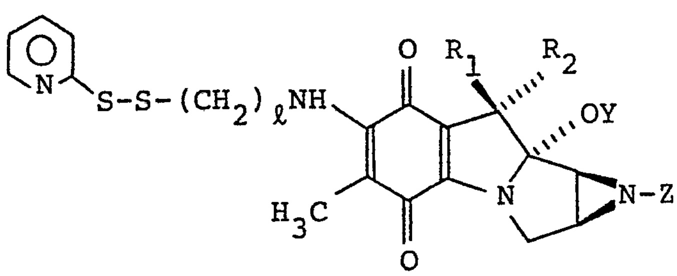

(I-1-1)

(wherein, ℓ, $R_1$, $R_2$, Y and Z are as defined above) can be prepared by reacting a compound represented by the formula (II):

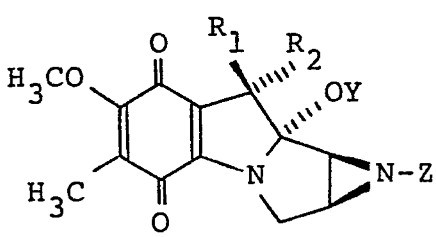

( II )

(wherein, $R_1$, $R_2$, Y and Z are as defined above) with a compound represented by the formula (III)

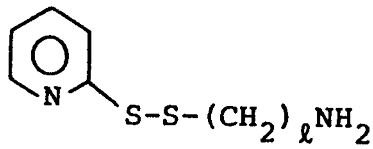

( III )

(wherein, $\ell$ is as defined above) or an acid adduct salt thereof in an inert solvent [under a basic condition when the acid adduct salt of the compound (III) is used].

As the inert solvent, chloroform, dichloromethane, methanol, ethanol, isopropanol, acetonitrile, etc. can be used alone or in a combination thereof. In case of an acid adduct salt of compound (III), a base is required to neutralize it, and the preferable base is an organic base such as triethylamine, pyridine, etc. It is preferable to add the base to the reaction system in an amount of at least twice the equivalent weight of the acid adduct salt of compound (III). The reaction temperature and reaction time are different depending on compound (III) or a solvent to be used, and usually are 0°C to 30°C and a few hours to a few days, respectively.

In the compounds (I-1), a compound represented by the formula (I-1-2):

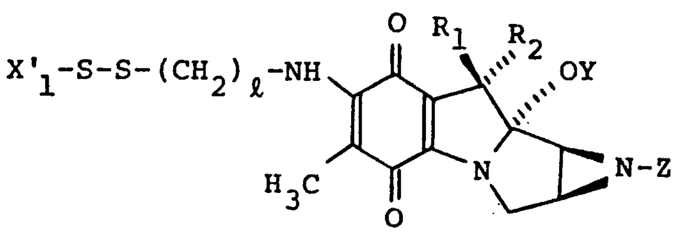

( I-1-2 )

(wherein $X'_1$ has the same meaning as $X_1$ in the formula (I-1) excluding 2-pyridyl group, and $\ell$, $R_1$, $R_2$, Y and Z are as defined above) can be prepared by reaction of the compound (I-1-1) with a compound represented by the formula (IV):

$$X'_1 - SH \qquad (IV)$$

 0214303

(wherein $X'_1$ is as defined above) in an inert solvent.

The solvent for use in the reaction includes halogenated lower alkanes such as chloroform, dichloromethane, etc., lower alkanols such as methanol, ethanol, isopropanol, etc., acetonitrile, dimethylformamide, dimethylsulfoxide, water, etc., and can be used alone or in a combination thereof. The reaction temperature and the reaction time are different depending on the compound (IV), and usually can be 0°C to 30°C and a few minutes to a few hours.

(2) In case of X being $X_2$ [compound (I) in this case is referred to as compound (I-2)]

Compound (I-2) can be prepared by reacting a compound represented by the formula (V):

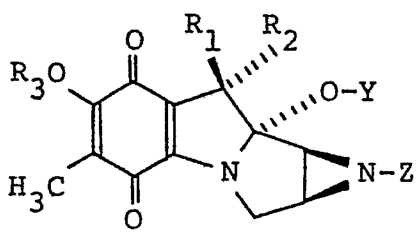

( V )

(wherein $R_3$ is a lower alkyl group, a lower alkanoyl group, or a benzoyl group; Y, Z, $R_1$ and $R_2$ are as defined above) with a compound represented by formula (VI):

$$H_2N-A_1-SS-A_1-NH_2 \qquad (VI)$$

(wherein $A_1$ is as defined above), or a compound represented by formula (VII):

$$HS-A_1-NH_2 \qquad (VII)$$

(wherein $A_1$ is as defined above), or an acid adduct salt of compound (VI) or (VII) in an inert solvent.

It seems that mitomycin represented by formula (VIII):

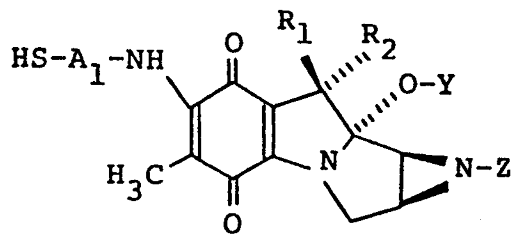

( VIII )

(wherein $A_1$, Y, Z, $R_1$ and $R_2$ are as defined above) is formed at first by the reaction of the compound (VII) with the compound (V). However, the compound (VIII) cannot be isolated, and the main product is the compound (I-2). It seems that, even if the compound (VIII) is formed, the quinone group in the molecule works as an oxidizing agent and oxidizes the thiol group into disulfide, where the quinone group must be converted to a dihydroxyphenyl group, but the dihydroxyphenyl group is much susceptible to air oxidation, and is reoxidized into the quinone nucleus during the reaction or during the post-treatment or purification, and that the compound (I-2) can finally be isolated as a stable product. To synthesize the compound (I-2) in higher yield, it is better to use the compound (VI) as a starting material than to use the compound (VII).

Examples of the compound (VI) include $[-S(CH_2)_2NH_2]_2$, $[-S(CH_2)_3NH_2]_2$, $[-S(CH_2)_4NH_2]_2$, $[-S(CH_2)_5NH_2]_2$, $[-S(CH_2)_6NH_2]_2$, $[-S(CH_2)_7NH_2]_2$, $[-S(CH_2)_8NH_2]_2$, $[-S(CH_2)_9NH_2]_2$, $[-S(CH_2)_{10}NH_2]_2$, $[-S(CH_2)_{11}NH_2]_2$, $[-S(CH_2)_{12}NH_2]_2$, $[-SCH_2CH_2OCH_2CH_2NH_2]_2$, $[-S(CH_2CH_2O)_2CH_2CH_2NH_2]_2$, $[-S(CH_2CH_2O)_3CH_2CH_2NH_2]_2$, $[-S(CH_2CH_2O)_4CH_2CH_2NH_2]_2$, $[-S(CH_2CH_2O)_5CH_2CH_2NH_2]_2$, etc.

The compound (VII) includes aminothiols corresponding to the reduction types of these disulfides. As a salt of compound (VI) or (VII), a hydrochloride is usually used, and a hydrobromide or a sulfate, or an organic acid salt such as acetate, paratoluenesulfonate, etc. may also be used. In case of using these salts as a starting material, it is desirable to add thereto a base in an amount necessary for neutralizing the acid liberated

during the reaction or in larger amount. To this end, a base that fails to react with the compound (V) or has a very low reaction rate in case of reaction is preferable. Suitable examples of the base include tertiary amines such as triethylamine, pyridine, etc., bicarbonates or carbonates of alkali metal or alkaline earth metal, etc.

The solvent for the reaction includes methanol, ethanol, isopropanol, chloroform, dichloromethane, acetonitrile, tetrahydrofuran, methylene glycol dimethyl ether, dimethylformamide, dimethylsulfoxide, water, etc., which can be used alone or in their mixture. Reaction temperature and time are different depending upon the species of compound (V) and compound (VI) or compound (VII), and are usually in a range of 0 to 30°C for a few hours to one day.

Post-treatment in the preparations of the compounds (I-1) and (I-2) is different depending on the solubility of reaction product. When the product is insoluble or sparingly soluble in water, the reaction solution is directly concentrated under reduced pressure, or extracted with chloroform or ethyl acetate, the extract is concentrated, and then purification is carried out. When the product is water-soluble, the reaction solution is directly led to a purification step. Alternatively, chloroform or ethyl acetate is added to the reaction solution, the solution is extracted with water, the aqueous layer is concentrated under reduced pressure, and then the concentrate is led to the purification step. In either case, purification is carried out by column chromatography, thin layer chromatography or recrystallization. The said procedures are disclosed in Japanese Published Unexamined Patent Application No. 104386/1984 and Japanese Published Unexamined Patent Application No. 175493/1984.

The compounds (I) have generally a distinguished antibacterial and anti-tumor activity, and its acute toxicity and bone-marrow toxicity are considerably reduced, compared with those of compounds whose $X = X_1$ and $\ell = 2$ or

those of compounds whose $X = X_2$ and $m = 2$ in the formula (I). Especially, compounds whose $X = X_1$ and $\ell = 4 - 8$, compounds whose $X = X_2$ and $m = 3 - 8$ and compounds whose $X = X_2$ and $n = 1 - 5$ respectively in the formula (I) are much improved even in CI value, which reflects selective toxicity against tumor and is shown in Experimental example 2, than compounds whose X = X, and $\ell = 2$ or compounds whose $X = X_2$ and $m = 2$ respectively in the formula (I).
Further, the compounds (I) wherein X is a residue of an amino acid having a thiol group or a dipeptide or tripeptide containing a residue of the amino acid and the residue being free from the thiol group, or X is a group represented by the formula:

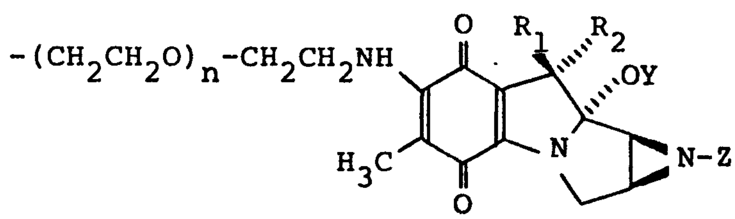

(wherein, n, $R_1$, $R_2$, Y and Z are as defined above) are much improved in water solubility.

The above-mentioned specific properties of the compounds (I) are demonstrated in the experimental examples.

<u>Experimental example 1</u>

Antibacterial activity of several compounds selected from compounds (I) according to the present invention are shown in Table 1 as minimum inhibitory concentration (μg/ml). The minimum inhibitory concentration was determined at pH 7.0 by agar dilution method. In Table 1, the symbols for bacteria have the following meanings.

SF <u>Streptococcus faecalis</u> ATCC 10541
SA <u>Staphilococcus aureus</u> ATCC 65389P
BS <u>Bacillus subtilis</u> 10707

PV *Proteus vulgaris* ATCC 6897
KP *Klebsiella penumoniae* ATCC 10031

Table 1

Antibacterial activity
(minimum inhibitory concentration, μg/ml)

| Compound | S F | S A | B S | P V | K P |
|---|---|---|---|---|---|
| 1 | 5 | 1.3 | 1.3 | 20 | 5 |
| 2 | >40 | >40 | 40 | | >40 |
| 3 | 5 | 10 | 2.5 | | |
| 4 | 0.78 | 0.2 | 0.39 | 1.6 | 0.2 |
| 6 | 1.6 | 0.78 | 0.78 | 6.3 | 1.6 |
| 7 | | 25 | 50 | | |
| 8 | 3.1 | 6.3 | 3.1 | 100 | 13 |
| 10 | 100 | 25 | 6.3 | 200 | 200 |
| 11 | 1.3 | 3.1 | 1.6 | 6.3 | 25 |
| 12 | 6.3 | 3.1 | 3.1 | 100 | 25 |
| 13 | 50 | 13 | 13 | 50 | 13 |
| 19 | 0.16 | 0.31 | <0.02 | 2.5 | 1.3 |
| 20 | 0.63 | 1.3 | 0.63 | 5 | 1.3 |
| 21 | 2.5 | 2.5 | 0.63 | 5 | 1.3 |
| 25 | 0.31 | 0.16 | 0.039 | 5 | 1.3 |
| 26 | 0.63 | 1.3 | 0.31 | 5 | 0.63 |
| 27 | 10 | 5 | 2.5 | 5 | 2.5 |
| 28 | 10 | 5 | 2.5 | 10 | 2.5 |
| 29 | 20 | 20 | 5 | 20 | 5 |

(Chemical structure of each compound is shown in later-described Table 4)

0214303

<u>Experimental example 2</u>

**Anti-tumor activity against Sarcoma 180 solid tumor and toxicity**

Anti-tumor activity ($ED_{50}$) against Sarcoma 180 solid tumor, acute toxicity ($LD_{50}$) and influence on peripheral leukocytes ($WBC_{4000}$) of several compounds selected from the compounds (I) are given in Table 2.

$WBC_{4000}$ shows a dosage of a medicament reducing the peripheral leukocytes to 4000/mm$^3$.

CI in the table shows chemotherapy index, and can be obtained according to $CI = LD_{50} / ED_{50}$. As is apparent from this definition, the CI value reflects the selective toxicity on the tumor, where a higher CI value means a better anti-tumor agent, and clinically means a broad acceptable range for medicament dosage. The CI value is one of the most important indices in the evaluation of anti-tumor agents.

In Table 8, data of anti-tumor activity, etc. of control compounds (the following four compounds with 2 methylene chains) are given together. These compounds can be represented as follows according to the representation of the formula (I).

| Compound | X | A | $R_1$ | $R_2$ | Y | Z |
|---|---|---|---|---|---|---|
| A | a | $(CH_2)_2$ | b | H | $CH_3$ | H |
| B | $CH_3CONH$- 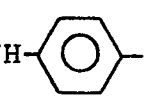 | " | " | " | " | " |
| C | $HOCH_2CH(OH)CH_2$- | " | " | " | " | " |
| D | $HO_2CCH_2NHCOCH(NH_2)CH_2$- | " | " | " | " | " |

a : 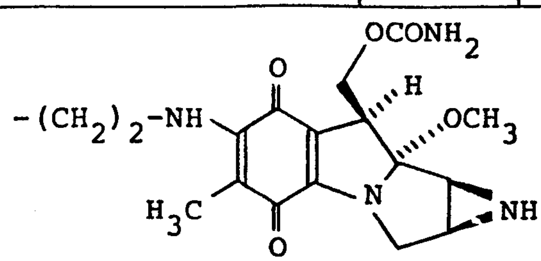

b : $-CH_2OCONH_2$

Compounds A, B, C and D are disclosed in Japanese Published Unexamined Patent Application Nos. 104386/1984, 175493/1984, 175493/1984 and 255789/1985, respectively.

Table 2

Anti-tumor activity and toxicity

| Compound | $LD_{50}$ (mg/kg) | $ED_{50}$ (mg/kg) | C I | $WBC_{4000}$ (mg/kg) |
|---|---|---|---|---|
| 1 | 90 | 25.4 | 3.5 | 99.7 |
| 4 | 200 | 32.6 | 6.1 | 118.5 |
| 6 | >200 | 26.9 | >7.4 | 88.3 |
| 8 | >200 | 34.6 | >5.7 | 42.3 |
| A | 18.8 | 6.3 | 3.0 | 16.6 |
| 25 | >100 | 43.7 | >2.3 | >100 |
| B | 30.0 | 8.5 | 3.5 | 14.2 |
| 20 | 65.6 | 59.7 | 1.1 | 53.1 |
| 26 | >100 | 51.0 | >2.0 | >100 |
| C | 22.5 | 12.5 | 1.8 | 20.8 |
| 21 | >100 | 33.1 | >3.0 | 93.9 |
| 28 | >200 | 32.0 | >6.3 | >200 |
| 29 | >200 | 28.5 | >7.0 | 55.4 |
| D | 27.0 | 9.6 | 2.8 | 20.5 |
| 12 | 75 | 17.6 | 4.3 | >50 |
| 13 | ≥100 | 20.8 | ≥4.8 | >100 |

Compounds 1, 4, 6 and 8 are different only in m from compound A, and have m = 3, 4, 5 and 6, respectively. Compound 25 is different only in ℓ from compound B, and has ℓ = 4. Compounds 20 and 26 are different only in ℓ from compound C, and have ℓ = 3 and 4, respectively. Compounds 21, 28 and 29 are different only in ℓ from compound D, and have ℓ = 3, 5 and 6, respectively.

It is apparent from Table 2 that the group of compounds 1, 4, 6 and 8 and the group of compounds 21, 28 and 29 have both considerably increased CI value and $WBC_{4000}$ than each control compound. Further, the group of compounds 20 and 26 has considerably increased $WBC_{4000}$ than the control compound, and compound 26 has increased CI value, too. Compound 25 has increased $WBC_{4000}$ than compound B. Further, it is apparent that the group of compounds 12 and 13 has very large values in both CI and $WBC_{4000}$.

The experiments were performed according to the following procedures.

(1) Effect against Sarcoma 180 solid tumor cells:

$5 \times 10^6$ cells of Sarcoma 180 solid tumor were intraperitoneally implanted into ddY mice. 7 days later, ascites cells were sampled. The cells were washed once with a sterilized physiological sodium chloride solution and were used to prepare a cell suspension containing $5 \times 10^7$ cells per ml with a sterilized physiological sodium chloride solution. On each occasion, 0.1 ml of the cell suspension was subcutaneously implanted into the right axilla of a male mouse (ddY strain; body weight 20 ± 2 g). The test compound was dissolved in a physiological sodium chloride solution with or without addition to Tween 80 and was administered into the tail vein of each mouse of a group consisting of 5 mice at a dose of 0.1 - 0.2 ml, 24 hours after the implantation of the tumor cells.

The anti-tumor activity was determined in the following manner. 7 days after the implantation, the major axis (a) and the minor axis (b) of the tumor were

measured to calculate a value of "a x $b^2/2$" which corresponds to the volume of the tumor. The anti-tumor activity was expressed by the ratio (T/C) of the volume (T) of the tumors of the group of animals administered with the test compound to the corresponding volume (C) of tumors of the untreated animals.

(2) Determination of $ED_{50}$:

$ED_{50}$ shows the amount of a substance needed for reducing the volume of Sarcome 180 solid tumors in mice to 50% on the basis of the corresponding volume of Sarcoma solid tumors in control animals.

On graph paper, T/C was indicated by an arithmetic scale on the longitudinal axis and the administered amount of the test compound was indicated by a logarithmic scale on the lateral axis. The relationship between the dose and T/C was shown by a straight line determined by the method of least squares, from which a dose corresponding to T/C of 0.5 was obtained.

(3) Acute toxicity:

Each animal of the test group consisting of 5 ddY mice was administered intraperitoneally once with a test compound. After the administration, the animals were observed for 14 days and deaths were noted. The $LD_{50}$ was determined by Beherns Kaerber's method.

(4) Effect on the peripheral leucocytes number:

Sarcoma 180 solid tumor cells (5 x $10^6$) were subcutaneously implanted into the right axilla of each mouse (body weight 20 ± 2 g) of a group consisting of 5 male mice (ddY strain). 24 hours after implantation, a test compound was intraperitoneally administered to each animal. 4 days after the administration, blood (each 0.02 ml) was collected from the suborbital plexus vein of each tumor-carrying animal. The collected sample of blood was dispersed in 9.98 ml of Cell-Kit Seven solution. One drop of saponin solution was added to the sample to

dissolve erythrocytes and then a microcell counter was used to count the number of leucocytes. On graph paper, the number of leucocytes was indicated by an arithmetic scale on the longitudinal axis and the dose of the test compound was indicated by a logarithmic scale on the lateral axis. The relationship between the number of peripheral leucocytes and the dose of the test compound was plotted and the dose (i.e., $WBC_{4000}$) corresponding to 4000 peripheral leucocytes/$mm^3$ (about half the number of leucocytes of normal mice) was obtained.

Experimental example 3 (Experiment of solubility)

1 mg of compound 12 was added to 100 μl of 0.03 M phosphate buffer (pH 7.0). The mixture was stirred at 22°C for 15 minutes and filtered through Ekicrodisc 3 (water system 0.45 μm-disposable filter unit, Gelman Sciences Japan Co.) to remove insoluble compound 12. The filtrate is a saturated solution of compound 12. Predetermined amount of the saturated solution was injected into a high performance liquid chromatograph [column: YMC-A212 ($C_8$) ∅ 6 mm, 150 mm, eluting solvent: 0.01 M-ammonium acetate/methanol = 4 : 6, wave length for detection: 254 nm, retention time: 17.76 minutes] and peak area of the chromatogram was calculated. Separately, a solution of the compound 12 in methanol having a certain concentration of the compound was used to make the calibration curve. The solubility of compound 12 under the above condition was calculated to be 290 μg/ml by the calibration curve.

Solubilities of compound 13 and compounds 6 and 10 as control compounds of compounds 12 and 13, respectively are measured in the same manner as described above. The result is shown in Table 3. As apparent from Table 3, compound 12 has a solubility of about 300 times that of compound 6, and compound 13 has a solubility of 530 μg/ml, whereas compound 10 is scarcely soluble in water, which made it impossible to determine the solubility.

Table 3

| Compound | Solubility (μg/ml) | Compound | Solubility (μg/ml) |
|---|---|---|---|
| 12 | 290 | 6 | 0.94 |
| 13 | 530 | 10 | - |

Compounds (I) have a distinguished antibacterial and anti-tumor activities and can be used as pharmaceutical composition such as an antibacterial agent, an anti-tumor agent, etc.

A compound (I) can be used as anti-tumor agent together with at least one of pharmaceutical diluents, adjuvants or carriers. For example, a compound (I) is dissolved in physiological saline solution, or glucose, lactose or mannit injection solution and is usually intravenously administered as injection to a mammal especially to homo sapiens at a dosage of 0.06 - 5 mg/kg. Further, intraarterial administration, intraperitoneal administration and administration into thoracic cavity can be conducted in the same dosage as above. The present anti-tumor agent can be freeze dried according to Japanese Pharmacopoeia, or can be made powder injection with addition of sodium chloride. Further, the anti-tumor agent can contain well known pharmacologically acceptable diluent, adjuvant and/or carrier such as salts satisfying the medicament uses. When used as an injection, it is sometimes preferable to use an additive for increase of the solubility together. Dosage can be appropriately adjusted in view of ages or symptoms. Administration schedule can also be changed by symptoms or dosages, and for example, an intermittent administration such as one administration in a week or three weeks can be adopted. The present anti-tumor agent can be orally or rectally administered in the same dosages. For the oral administration, it can be administered in the form of tablet,

0214303

powder, granules, syrups or suppository with an appropriate filler.

The present invention also relates to disulfide compounds represented by the formula (IX):

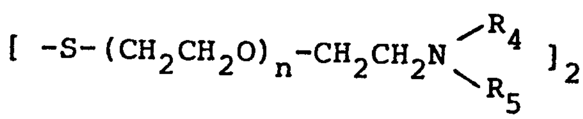

( IX )

(wherein, n is as defined above, and $R_4$ and $R_5$ are both hydrogen atoms or combined to represent a phthaloyl group) and acid adduct salts thereof. Compounds (IX) and acid adduct salts thereof are useful as intermediates for synthesis of compound (I) wherein X is a group represented by the formula:

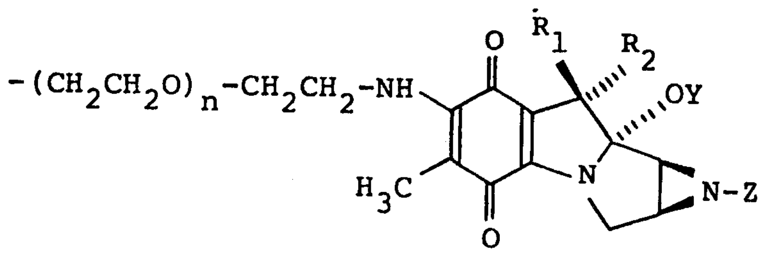

The acid adduct salts of compound (IX) include salts with various inorganic acids such as hydrochlorides, hydrobromides, hydroiodides, nitrates, sulfates, phosphates, etc., and salts with various organic acids such as formates, acetates, propionates, benzoates, citrates, succinates, oxalates, methanesulfonates, ethanesulfonates, propanesulfonates, 1,2-ethanedisulfonates, benzenesulfonates, p-toluenesulfonates, etc.

Compound (IX) and its acid adduct salt can be synthesized according to the following steps:

Step 1.

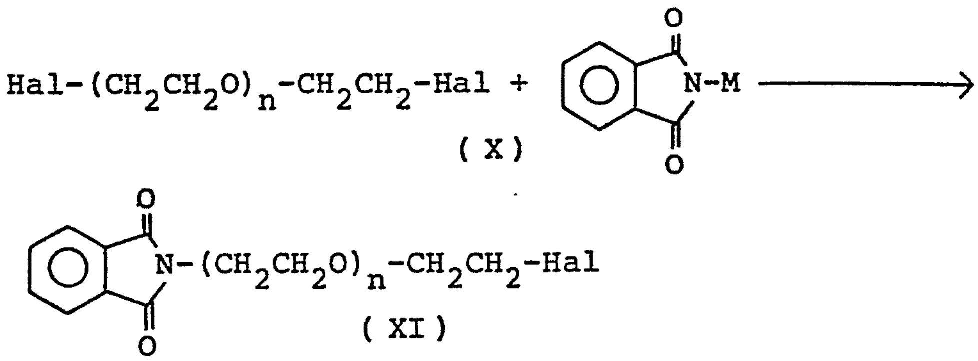

( X )

( XI )

Step 2.

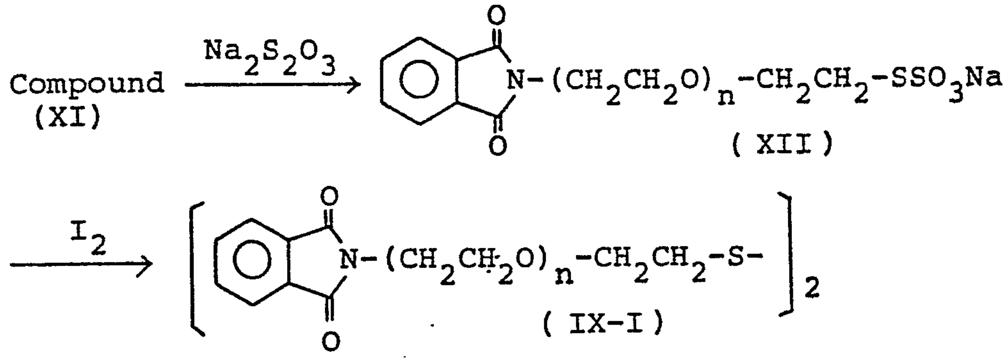


( XII )

( IX-I )

Step 3.

Compound (IX-1) $\xrightarrow{N_2N-NH_2}$ $[H_2N-(CH_2CH_2O)_n-CH_2CH_2S-]_2$

( IX-2 )

$\xrightarrow{\text{Acid}}$ An acid adduct salt of compound (IX-2)

(wherein, Hal represents a halogen atom and M represents an alkali metal, and n is as defined above).

In the foregoing definition, the halogen atom includes chlorine, bromine, etc., and the alkali metal includes sodium, potassium, etc.

The individual steps will be described below:

Step 1:

Compound (X) is reacted with an alkali metal salt of phthalimide in an inert solvent to prepare compound (XI).

A suitable inert solvent is acetone, dimethylformamide, etc., and they can be used in a mixture, if necessary. Reaction temperature and reaction time are different depending upon the species of compound (X) and the solvent, and are usually in a range of room temperature to 100°C and for a few hours to a few days. To obtain compound (XI) in high yield, it is preferable to use 1.5 to 2.0 equivalent weights of compound (X). After the reaction, by-produced inorganic salts, etc. are removed from the reaction product solution by filtration, and the filtrate is concentrated and then purified by recrystallization from an appropriate solvent, by column chromatography, etc.

Step 2:

The compound (XI) obtained in Step (I) is dissolved in water or in a solvent mixture of water and alcohol such as methanol, ethanol, etc., and subjected to reaction with one equivalent weight of sodium thiosulfate. Reaction temperature is 50 to 80°C, and reaction time may be a few hours. Progress of reaction can be checked by tracing the decrease in the amount of compound (XI) by thin layer chromatography. The reaction product is a Bunte salt [compound (XII)]. After compound (XI) has disappeared, one equivalent weight of iodine, as dissolved in methanol or ethanol, is added thereto. Reaction is again traced by thin layer chromatography. After the disappearance of compound (XII) and formation of compound (IX-1) have been confirmed, the solvent is removed therefrom by distillation, and the residue is purified by recrystallization or by column chromatography, whereby compound (IX-1) is obtained.

Step 3:

Compound (IX-1) is reacted with 2 equivalent weights of hydrazine to obtain compound (IX-2) freed from the phthalimido group. As the reaction solvent, alcohol such as methanol, ethanol, etc. are used. Reaction temperature can be the boiling point of the solvent used, and

reaction time can be a few fours. After the reaction, the reaction solution is acidified with an acid such as hydrochloric acid, and the formed salt of phthalhydrazine is allowed to precipitate and removed by filtration. The filtrate is diluted with water, and neutral substances such as unreacted substances, etc. are extracted with an organic solvent such as chloroform, etc., and removed.

The aqueous layer is made alkaline with NaOH, etc. and extracted with an organic solvent such as chloroform, etc. The extract is washed with water, and dried, and compound (IX-2) is obtained by removing the solvent therefrom. The organic layer containing compound (IX-2) extracted from the alkaline solution is back extracted with dilute hydrochloric acid, and the aqueous layer is concentrated, or compound (IX-2), as in a solution of alcohol, etc., is concentrated after addition of more than the equivalent weight of dilute hydrochloric acid for the neutralization thereto, whereby dihydrochloride of compound (IX-2) is obtained. When other salt is required, the acid to be used at the said final stage must be changed. Salt of compound (IX-2) thus obtained can be further purified by recrystallization.

The compound (IX-1) fails to take the form of acid adduct salt, and thus the acid adduct salt of compound (IX) substantially means an acid adduct salt of compound (IX-2).

## Best Mode for Carrying Out the Invention

Physico-chemical data shown in the following Examples were obtained by means of the following instruments.

$^1$H-NMR : Nihon Denshi FX-100 (100 MHz), Bruker AM400 (400 MHz), and Varian EM-390 (90 MHz). Measurement was carried out in pyridine-d5, unless otherwise particularly mentioned, and the peak integral intensity in symmetrical disulfide compounds, e.g. compounds

1 - 13 was shown as a relative value (the actual number of hydrogen atoms was its double).

IR : Shimazu IR-G-27. Measurement was carried out according to KBr method, unless otherwise particularly mentioned.

MS : Hitachi M-80B

TLC : Merck Art 5714 (silica gel plate)

M.P : Yanagimoto micro melting point measuring apparatus (hot plate system).

The found value by elemental analysis of mitomycin derivatives is often not in accordance with the calculated value, and this tendency is also pronounced in the present compounds, and thus the found value fails to reflect the molecular formula simply, and will not be described. A substitute for the elemental analysis is an MS spectrum (mass spectrum), but in case of mitomycin derivatives, $M^+$ is generally hard to observe, and, when observed, its intensity is often weak. $M^+$ of the present compounds was not observable under the measurement conditions of the ordinary EI method. As a result of various investigations, peaks reflecting molecular weight could be observed according to the SIMS method. However, the peak observed according to the method did not correspond to $M^+$, but to $[M+1]^+$, $[M+2]^+$ and $[M+3]^+$, and in the symmetrical disulfide compounds $[M+4]^+$ or $[M+5]^+$ besides $[M+1]^+$, $[M+2]^+$ and $[M+3]^+$ were observed, and some of these were observed at the same time in every case. The reasons seem as follows: in the SIMS method, a sample mixed with glycerine is ionized by bombardment of $Xe^+$, and the quinone moiety of mitomycin is reduced by the hydrogen radicals generated from the glycerine at that time. Some of the compounds of the present invention have two mitomycin skeletons for each, and in there compounds, maximum 4 hydrogens are taken into one molecule. That is, there is a possibility of conversion to a compound having two

hydroquinone skeletons in the mass spectrometer. In that case, the mass number will be increased by 4, but addition of hydrogen ions or hydrogen radicals may also take place, as often observable in the ordinary mass spectrum. Thus, it can happen that $[M+5]^+$ ion is observed in the spectrum. For example, in case of mitomycin C having the molecular weight of 334, peaks are observable at m/z 335, 336 and 337 according to the SIMS method, where it can be explained that the peak at m/z 337 is due to the ion resulting from reduction of quinone part and further addition of hydrogen. The molecular weights of compounds shown in Examples are not directly proved by MS for the foregoing reasons, but in a group of such special compounds, the appearance of peaks $[M+1]^+$ to $[M+5]^+$ seems strongly to support the individual molecular weights and molecular formulae. It is also well-known that a compound (V) can undergo substitution reaction with a primary amine selectively at the 7-position, and it is clearly shown by $^1$H-NMR that the product is a compound (I). Thus, the molecular formulae of the individual compounds are believed to be well proved thereby.

The powders of compounds (I) are all dark as if called black, but in Examples the individual color tones are described. The structures of the compounds disclosed in Examples are shown in Table 4, as corresponded to formula (I).

Table 4

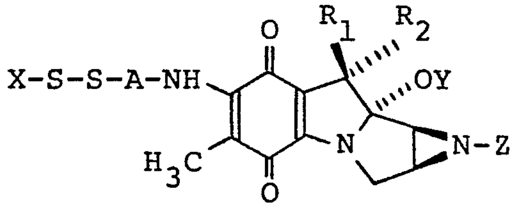

( I )

| Compound | X | A | $R_1$ | $R_2$ | Y | Z |
|---|---|---|---|---|---|---|
| 1 | $X_2$ | $(CH_2)_3$ | b | H | $CH_3$ | H |
| 2 | " | " | H | b | H | $CH_3$ |
| 3 | " | " | $=CH_2$ | | " | " |
| 4 | " | $(CH_2)_4$ | b | H | $CH_3$ | H |
| 5 | " | " | H | b | H | $CH_3$ |
| 6 | " | $(CH_2)_5$ | b | H | $CH_3$ | H |
| 7 | " | " | H | b | H | $CH_3$ |
| 8 | " | $(CH_2)_6$ | b | H | $CH_3$ | H |
| 9 | " | " | H | b | H | $CH_3$ |
| 10 | " | $(CH_2)_8$ | b | H | $CH_3$ | H |
| 11 | " | $(CH_2)_{12}$ | " | " | " | " |
| 12 | " | c | " | " | " | " |
| 13 | " | d | " | " | " | " |
| 14 | 2-pyridyl | $(CH_2)_3$ | " | " | " | " |
| 15 | " | " | H | b | H | $CH_3$ |
| 16 | " | $(CH_2)_4$ | b | H | $CH_3$ | H |
| 17 | " | $(CH_2)_5$ | " | " | " | " |
| 18 | " | $(CH_2)_6$ | " | " | " | " |
| 19 | $CH_3CONH$- 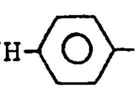 | $(CH_2)_3$ | " | " | " | " |
| 20 | $HOCH_2CH(OH)CH_2$- | " | " | " | " | " |
| 21 | $HO_2CCH_2NHCOCH(NH_2)CH_2$- | " | " | " | " | " |
| 22 | $CH_3CONH(CH_2)_2$- | " | H | b | H | $CH_3$ |
| 23 | $CH_3CH_2$- | " | " | " | " | " |
| 24 | 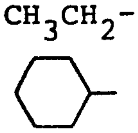 | " | " | " | " | " |

| Compound | X | A | $R_1$ | $R_2$ | Y | Z |
|---|---|---|---|---|---|---|
| 25 | $CH_3CONH$- 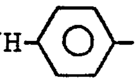 - | $(CH_2)_4$ | b | H | $CH_3$ | H |
| 26 | $HOCH_2CH(OH)CH_2$- | " | " | " | " | " |
| 27 | $HO_2CCH_2NHCOCH(NH_2)CH_2$- | " | " | " | " | " |
| 28 | " | $(CH_2)_5$ | " | " | " | " |
| 29 | " | $(CH_2)_6$ | " | " | " | " |

$X_2$ : 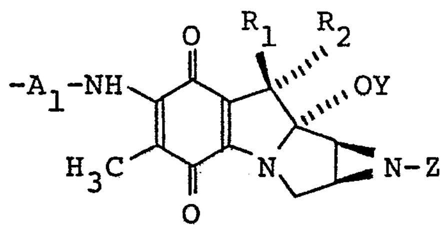

It is noted that when $X = X_2$, $A = A_1$

b : $-CH_2OCONH_2$

c : $-CH_2CH_2OCH_2CH_2-$

d : $-(CH_2CH_2O)_2CH_2CH_2-$

Example 1

7-N,7'-N'-(dithioditrimethylene) dimitomycin C (Compound 1)

At first, 175 mg of mitomycin A was dissolved in 4 ml of methanol, and 104.5 μl of triethylamine and 63.3 mg of homocystamine dihydrochloride were added thereto. The mixture was stirred at room temperature for 12 hours. After the reaction, the solvent was removed therefrom by distillation under reduced pressure, and the residue was subjected to column chromatography using 15 g of silica gel. Blue fraction eluted by chloroform / methanol (9 : 1 V/V) was recovered and concentrated, and, after addition of n-hexane thereto, reconcentrated again and dried under reduced

pressure, whereby 118 mg of Compound 1 was obtained (yield: 58%).

M.P.: 122 - 135°C (not definitely determined due to the dark color of the sample; the measurement of M.P. which follows was also under the similar situation).

$^1$H-NMR (100 MHz): δ 2.00(2H, quint, J=7.1), 2.14(3H, s), about 2.7(1H, b), 2.80(2H, t, J=7.1), 3.14 (1H, bs), 3.22(3H, s), 3.61(1H, bd, J=12.7), 3.66(2H, q, J=6.7), 4.00(1H, dd, J=11.0, 4.4), 4.54(1H, d, J=12.7), 5.04(1H, t, J=10.6), 5.38 (1H, dd, J=10.3, 4.4), 7.08(1H, t, J=7.0), 7.63(2H, bs)

IR : 3300, 2940, 1716, 1631, 1550, 1509, 1445, 1328, 1059 $cm^{-1}$

MS(EI) : m/z 407($M^+$/2, $C_{36}H_{46}N_8O_{10}S_2$ = 814)

MS(SIMS) : m/z 816 [$(M+2)^+$], 818 [$(M+4)^+$]

TLC : Rf = 0.35 (chloroform / methanol, 9 : 1 V/V)

Example 2

7-N,7'-N'-(dithioditrimethylene) dimitomycin C (Compound 1)

At first, 22 mg of mitomycin A was dissolved in 0.6 ml of methanol, and 8.8 μl of triethylamine and 7.7 mg of homocysteamine monohydrochloride were added thereto. The mixture was stirred at room temperature for 4 hours. When the reaction was traced according to TLC using chloroform / methanol (9 : 1 V/V) and ethyl acetate / methanol (9 : 1 V/V) as developing solvents, formation of a compound having the same dark bluish violet color and the same Rf value as those of the compound obtained in Example 1 was confirmed. However, many by-products having yellow and yellowish orange colors were produced at the same time.

Example 3

7-N,7'-N'(dithioditrimethylene) dimitomycin D (Compound 2)

Reaction was carried out in the same manner as in Example 1 except that 175 mg of mitomycin B was used in

place of mitomycin A, whereby 121 mg of Compound 2 was obtained as dark bluish green powder (yield: 59%).

M.P.: 153 - 157°C

$^1$H-NMR (100 MHz): δ 1.93(2H, quint, J=7.1), 2.10(3H, s), 2.13(3H, s), 2.23(1H, dd, J=4.9, 1.7), 2.47 (1H, d, J=4.9), 2.74(2H, t, J=7.1), 3.59(2H, q, J=7.1), 3.67(1H, dd, J=12.9, 1.7), 4.22(1H, dd, J=9.8, 3.7), 4.45(1H, d, J=12.9), 5.20(1H, t, J=10.1), 5.47(1H, dd, J=10.5, 3.7), 7.07(1H, t, J=6.6), 7.47(2H, bs), 8.32(1H, bs)

IR: 3300, 2955, 1710, 1631, 1547, 1510, 1450, 1331, 1054 $cm^{-1}$

TLC: Rf = 0.20 (chloroform / methanol, 9 : 1 V/V)

MS (EI): m/z 407 (1/2 $M^+$, $C_{36}H_{46}N_8O_{10}S_2$ = 814)

MS (SIMS): m/z 816 [$(M+2)^+$], 817 [$(M+3)^+$)], 819 [$(M+5)^+$)]

Example 4

7-N,7'-N'-(dithioditrimethylene)bis-9a-O-demethylmitomycin G (Compound 3)

Reaction was carried out in the same manner as in Example 1, using 144 mg of mitomycin H, 104.5 µl of triethylamine and 63.3 mg of homocystamine dihydrochloride. The reaction product solution was diluted with 200 ml of chloroform, and washed twice with 20 ml of 0.1 M phosphate buffer (pH 5.0). The chloroform layer was further washed with water and an aqueous saturated sodium chloride solution, and then dried over anhydrous sodium sulfate. After removal of the drying agent by filtration, the solvent was removed therefrom by distillation under reduced pressure, and the residue was subjected to column chromatography using 50 g of silica gel and chloroform / acetone (7 : 3 V/V) as an eluting solvent. Blue fractions were collected, concentrated under reduced pressure, and, after further addition of n-hexane thereto, concentrated to dryness, whereby 109 mg of Compound 3 was obtained as dark yellowish brown powder (yield: 63%).

M.P.: no definite melting point was shown, and the color was slowly changed into dark brown during heating from 140°C to 250°C.

$^1$H-NMR (100 MHz): δ 1.92(2H, quint, J=6.7), 2.07(3H, s), 2.15(3H, s), 2.28(1H, dd, J=4.6, 1.7), 2.62 (1H, d, J=4.6), 2.72(2H, t, J=6.8), 3.57(2H, q, J=6.8), 3.75(1H, dd, J=12.9, 1.7), 4.74(1H, d, J=12.7), 5.81(1H, d, J=1.2), 6.48(1H, d, J=1.2), 7.09(1H, t, J=6.4)

IR : 3295, 2950, 1640, 1509, 1443, 1204, 1049 $cm^{-1}$

TLC : Rf = 0.23 (chloroform / acetone, 7 : 3 V/V)

MS (EI) : m/z 346 ($M^+/2$, $C_{34}H_{40}N_6O_6S_2$ = 692)

MS (SIMS) : m/z 693 [$(M+1)^+$], 695 [$(M+3)^+$], 696 [$(M+4)^+$]

Example 5

7-N,7'-N'-(dithioditetramethylene) dimitomycin C (Compound 4)

At first, 175 mg of mitomycin A was dissolved in 4 ml of methanol, and 104.5 μl of triethylamine and 70.3 mg of 4,4'-dithiodibutylamine dihydrochloride were added thereto. The mixture was stirred at room temperature for 5.5 hours, and then left standing in a refrigerator at 5°C overnight. Then, the mixture was diluted with 100 ml of ethyl acetate, and washed with an aqueous sodium bicarbonate solution. The aqueous layer was extracted with 50 ml of ethyl acetate, and both ethyl acetate layers were mixed together, washed with water and then with an aqueous saturated sodium chloride solution, and dried over anhydrous sodium sulfate. After removal of the drying agent by filtration, the solvent was removed therefrom by concentration under reduced pressure, and the residue was subjected to silica gel column chromatography using chlorform / methanol (93 : 7 V/V) as an eluting solvent. Blue band fractions were collected and concentrated, and, after addition of n-hexane thereto, the solvent was once more removed therefrom by distillation under reduced pressure.

The residue was concentrated to dryness, whereby 205 mg of Compound 4 was obtained as dark green powder (yield: 97%).

M.P.: 126 - 134°C

$^1$H-NMR (100 MHz): δ 1.72(4H, m), 2.14(3H, s), 2.76 (2H, t, J=6.6), about 2.8(1H, br), 3.15(1H, bs), 3.23(3H, s), 3.53(2H, q, J=6.4), 3.61(1H, bd, J=12.7), 4.00(1H, dd, J=11.0, 4.4), 4.56(1H, d, J=12.7), 5.03(1H, t, J=10.6), 5.38(1H, dd, J=10.5, 4.4), 7.02(1H, t, J=6.4), 7.62(2H, bs)

IR : 3310, 2950, 1720, 1636, 1553, 1510, 1448, 1329, 1059 $cm^{-1}$

TLC : Rf = 0.34 (chloroform / methanol, 9/1 V/V)

MS (SIMS): m/z 843 [$(M+1)^+$], 844 [$(M+2)^+$], 845 [$(M+3)^+$], $C_{38}H_{50}N_8O_{10}S_2$ = 842

Example 6

7-N,7'-N'-(dithioditetramethylene) dimitomycin D (Compound 5)

Reaction was carried out in the same manner as in Example 5, using 175 mg of mitomycin B, 104.5 μl of triethylamine and 70.3 mg of 4,4'-dithiodibutylamine dihydrochloride, whereby 187 mg of Compound 5 was obtained as dark green powder (yield: 89%) [chloroform / methanol (9 : 1 V/V) was used as an eluting solvent in the silica gel column chromatography].

M.P.: 154 - 161°C

$^1$H-NMR (100 MHz): δ 1.63(4H, br), 2.09(3H, s), 2.13 (3H, s), 2.23(1H, dd, J=4.6, 1.7), 2.47(1H, d, J=4.6), 2.72(2H, t, J=6.3), 3.47(2H, q, J=6.4), 3.68(1H, dd, J=12.9, 1.7), 4.22(1H, dd, J=9.8, 3.7), 4.46(1H, d, J=12.9), 5.20(1H, t, J=10.2), 5.47(1H, dd, J=10.5, 3.7), 7.01(1H, t, J=6.4), 7.47(2H, s), 8.33(1H, bs)

IR : 3320, 2960, 1718, 1637, 1514, 1453, 1332, 1059 $cm^{-1}$

TLC : Rf = 0.26 (chloroform / methanol, 9 : 1 V/V)

MS (SIMS): m/z 844 [$(M+2)^+$], 845 [$(M+3)^+$], 846 [$(M+4)^+$], $C_{38}H_{50}N_8O_{10}S_2$ = 842

Example 7

7-N,7'-N'-(dithiodipentamethylene) dimitomycin C (Compound 6)

Reaction was carried out in the same manner as in Example 5, using 175 mg of mitomycin A, 104.5 μl of triethylamine and 77.3 mg of 5,5'-dithiodipentylamine dihydrochloride, whereby 207 mg of Compound 6 was obtained as dark bluish violet powder (yield: 95%).

M.P.: 123 - 128°C

$^1$H-NMR (100 MHz): δ 1.3-1.8(6H, m), 2.14(3H, s), 2.74(2H, t, J=6.6), about 2.8(1H, m), 3.14(1H, bs), 3.23(3H, s), 3.49(2H, q, J=6.6), 3.61(1H, dd, J=12.7, 1.7), 4.00(1H, dd, J=10.7, 4.4), 4.56(1H, d, J=12.7), 5.03(1H, t, J=10.5), 5.39 (1H, dd, J=10.3, 4.4), 6.96(1H, t, J=6.4), 7.60(2H, bs)

IR : 3300, 2940, 1720, 1632, 1551, 1509, 1444, 1328, 1057 $cm^{-1}$

TLC : Rf = 0.39 (chloroform / methanol, 9 : 1 V/V)

MS (SIMS): m/z 871 [$(M+1)^+$], 872 [$(M+2)^+$], 875 [$(M+5)^+$], $C_{40}H_{54}N_8O_{10}S_2$ = 870

Example 8

7-N,7'-N'-(dithiodipentamethylene) dimitomycin D (Compound 7)

Reaction was carried out in the same manner as in Example 6, using 175 mg of mitomycin B, 104.5 μl of triethylamine and 77.3 mg of 5,5'-dithiopentylamine dihydrochlroide, whereby 208 mg of Compound 7 was obtained as dark green powder (yield: 96%).

M.P.: 128 - 138°C

$^1$H-NMR (100 MHz): δ 1.3-1.8(6H, m), 2.09(3H, s), 2.13(3H, s), 2.23(1H, dd, J=4.6, 1.7), 2.47(1H, d, J=4.6), 2.72(2H, t, J=6.8), 3.42(2H, q, J=6.3),

3.68(1H, dd, J=12.9, 1.7), 4.22(1H, dd, J=9.8, 3.7), 4.47(1H, d, J=12.9), 5.21(1H, t, J=10.2), 5.48(1H, dd, J=10.5, 3.7), 6.96(1H, t, J=6.3), 7.47(2H, bs), 8.33(1H, bs)

IR : 3265, 2910, 1710, 1627, 1506, 1444, 1326, 1050 $cm^{-1}$

TLC : Rf = 0.30 (chloroform / methanol, 9 : 1 V/V)

MS (SIMS): m/z 871 [$(M+1)^+$], 872 [$(M+2)^+$], 873 [$(M+3)^+$], 874 [$(M+4)^+$], 875 [$(M+5)^+$]

$C_{40}H_{54}N_8O_{10}S_2$ = 870

Example 9

7-N,7'-N'-(dithiodihexamethylene) dimitomycin C (Compound 8)

Reaction was carried out in the same manner as in Example 5, using 175 mg of mitomycin A, 104.5 mg of triethylamine and 84.4 mg of 6,6'-dithiodihexylamine dihydrochloride, whereby 213 mg of Compound 8 was obtained as dark bluish violet powder (yield: 95%).

M.P.: 106 - 113°C

$^1$H-NMR (100 MHz): δ 1.2-1.8(8H, m), 2.15(3H, s), 2.76(2H, b, J=6.6), about 2.8(1H, bm), 3.15(1H, d, J=4.4), 3.23(3H, s), 3.48(2H, q, J=6.6), 3.62 (1H, dd, J=12.7, 1.7), 4.00(1H, dd, J=11.0, 4.4), 4.57(1H, d, J=12.7), 5.04(1H, t, J=10.6), 5.40 (1H, dd, J=10.3, 4.4), 6.96(1H, t, J=6.6), 7.61(2H, bs)

IR : 3290, 2910, 1713, 1628, 1548, 1504, 1441, 1320, 1052 $cm^{-1}$

TLC : Rf = 0.48 (chloroform / methanol, 9 : 1 V/V)

MS (EI): m/z 499 ($M^+$/2, $C_{42}H_{58}N_8O_{10}S_2$ = 898

MS (SIMS): m/z 900 [$(M+2)^+$], 901 [$(M+3)^+$]

Example 10

7-N,7'-N'-(dithiodihexamethylene) dimitomycin D (Compound 9)

Reaction was carried out in the same manner as in Example 6, using 175 mg of mitomycin B, 104.5 μl of triethylamine and 84.4 mg of 6,6'-dithiohexylamine dihydrochloride, whereby 211 mg of Compound 9 was obtained as dark bluish green powder (yield: 94%).

M.P.: 131 - 135°C

$^1$H-NMR (100 MHz): δ 1.2-1.8(8H, m), 2.11(3H, s), 2.13(3H, s), 2.24(1H, dd, J=4.9, 2.0), 2.48(1H, d, J=4.9), 2.75(2H, t, J=6.8), 3.41(2H, q, J=6.6), 3.69(1H, dd, J=12.9, 2.0), 4.23(1H, dd, J=9.8, 3.7), 4.48(1H, d, J=12.9), 5.22(1H, t, J=10.2), 5.49(1H, dd, J=10.5, 3.7), 6.95(1H, t, J=6.6), 7.48(2H, bs), 8.35(1H, bs)

IR : 3290, 2930, 1715, 1630, 1543, 1506, 1448, 1327, 1052 $cm^{-1}$

TLC : Rf = 0.39 (chloroform / methanol, 9 : 1 V/V)

MS (EI): m/z 406 ($M^+$/2-HNCO)

MS (SIMS): m/z 900 [$(M+2)^+$], 901 [$(M+3)^+$], 903 [$(M+5)^+$], $C_{42}H_{58}N_8O_{10}S_2$ = 898

Example 11

7-N,7'-N'-(dithiodioctamethylene) dimitomycin C (Compound 10)

At first, 175 mg of mitomycin A was dissolved in a solvent mixture of 10 ml of methanol and 5 ml of chloroform and 104.5 μl of triethylamine and 98.3 mg of 8,8'-dithiodioctylamine dihydrochloride were added thereto. The mixture was stirred at room temperature and, 7.5 hours thereafter, 69.7 μl of triethylamine and 9.8 mg of 8,8'-dithiooctylamine dihydrochloride were further added thereto. Then, the mixture was stirred overnight. The reaction product solution was diluted with 100 ml of ethyl acetate and washed with an aqueous sodium bicarbonate solution. The aqueous layer was extracted with 50 ml of

acetic acid, and the organic layers were joined together. The mixture was washed with water and then with an aqueous saturated sodium chloride solution, and dried over anhydrous sodium sulfate.

After removal of the drying agent by filtration, the solvent was removed therefrom under reduced pressure, and the residue was subjected to column chromatography using 75 g of silica gel and chloroform / methanol (92 : 8 V/V). Blue fractions were collected and the solvent was removed therefrom under reduced pressure. After addition of n-hexane thereto, the operation was repeated, whereby 211 mg of Compound 10 was obtained as dark bluish violet powder (yield: 88%).

M.P.: Color was changed at 93 to 98°C, and furthermore changed even at 102 to 108°C.

$^1$H-NMR (100 MHz): δ 1.2-1.8(12H, m), 2.16(3H, s), 2.79(2H, t, J=6.8), about 2.8(1H, bm), 3.11(1H, bd, J=4.9), 3.23(3H, s), 3.48(2H, q, J=6.4), 3.60(1H, bd, J=12.7), 4.01(1H, dd, J=11.0, 4.4), 4.58(1H, d, J=12.7), 5.07(1H, t, J=10.8), 5.41 (1H, dd, J=10.3, 4.4), 6.97(1H, t, J=6.4), 7.62(2H, bs)

IR : 3300, 2928, 1719, 1639, 1558, 1518, 1450, 1330, 1060 $cm^{-1}$

TLC : Rf = 0.36 (chloroform / methanol, 9 : 1 V/V)

MS (EI): m/z 434 ($M^+$/2-HNCO)

MS (SIMS): m/z 959 [$(M+5)^+$], $C_{46}H_{66}N_8O_{10}S_2$ = 954

Example 12

7-N,7'-N'-(dithiodidodecamethylene) dimitomycin C (Compound 11)

At first, 150.8 mg of mitomycin A was dissolved in 10 ml of methanol, and 301 μl of triethylamine and 200 mg of crude 12,12'-dithiododecylamine dihydrobromide (containing about 72 mg of phthalic acid) were added thereto. The mixture was stirred at room temperature for 40 minutes, and, after addition of 5 ml of chloroform thereto, further

stirred overnight. After dilution with 100 ml of ethyl acetate, the mixture was washed with an aqueous saturated sodium bicarbonate solution. The washing solution was extracted twice with 50 ml of ethyl acetate, and all the ethyl acetate layers were joined together, washed with an aqueous saturated sodium bicarbonate solution, and then with an aqueous saturated sodium chloride solution, and dried over anhydrous sodium sulfate. After removal of the sodium sulfate therefrom by filtration, the solvent was removed therefrom by distillation under reduced pressure, the residue was subjected to column chromatography using 50 g of silica gel. Blue fractions eluted with chloroform / methanol (93 : 7 V/V) were collected, and concentrated under reduced pressure, and, after addition of n-hexane thereto, further concentrated to dryness under reduced pressure, whereby 165 mg of Compound 11 was obtained as dark bluish violet powder (yield: 66%).

M.P.: Color was changed at 88 to 102°C, and further changed even at 147°C, but no melting point was observed.

$^1$H-NMR (100 MHz): δ 1.2-1.9(20H, m), 2.17(3H, s), 2.81(2H, t, J=6.6), about 2.8(1H, bs), 3.11(1H, bd, J=4.4), 3.23(3H, s), 3.50(2H, q, J=6.6), 3.61(1H, bd, J=12.9), 4.01(1H, dd, J=11.0, 4.4), 4.58(1H, d, J=12.9), 5.07(1H, t, J=10.8), 5.41 (1H, dd, J=10.5, 4.4), 6.98(1H, t, J=6.1), 7.63 (2H, bs)

IR : 3310, 2928, 1720, 1640, 1560, 1514, 1452, 1328, 1060 $cm^{-1}$

TLC : Rf = 0.22 (chloroform / methanol, 95 : 5 V/V)

MS (SIMS): m/z 1071 [$(M+5)^+$], $C_{54}H_{82}N_8O_{10}S_2$ = 1066

Example 13

7-N,7'-N'-[dithiobis(3-oxapentamethylene)] dimitomycin C (Compound 12)

At first, 175 mg of mitomycin A was dissolved in 10 ml of methanol, and 348 ml of triethylamine and 78.3 mg

of 5,5'-dithiobis-3-oxapentylamine dihydrochloride (see Example 32 for the process for preparing this compound) were added thereto. The mixture was stirred at room temperature, and, 3 hours thereafter, 9.4 mg of said amine dihydrochloride was further added thereto. The mixture was further stirred overnight. Then, the mixture was diluted with 100 ml of ethyl acetate, and washed with an aqueous saturated sodium bicarbonate solution. The washing solution was extracted with 50 ml of ethyl acetate, and all the organic layers were joined together, washed with an aqueous saturated sodium bicarbonate solution and then with an aqueous saturated sodium chloride solution, and dried over anhydrous sodium sulfate. The drying agent was removed therefrom by distillation under reduced pressure, and the residue was subjected to column chromatography using 75 g of silica gel. Blue fractions eluted with chloroform / acetone / methanol (6 : 3 : 1 V/V) were collected, and the solvent was removed therefrom by distillation under reduced pressure. Then, n-hexane was added thereto, and the mixture was concentrated to dryness under reduced pressure, whereby 213 mg of Compound 12 was obtained as dark bluish violet powder (yield: 97%).

M.P.: 85 - 95°C

$^1$H-NMR (400 MHz): δ 2.09(1H, bs), 2.12(3H, s), 2.74 (1H, bs), 3.02(2H, t, J=6.4), 3.14(1H, bs), 3.22(3H, s), 3.58(1H, bd, J=12.7), 3.66(2H, t, J=5.4), 3.77(2H, q, J=5.7), 3.79(2H, t, J=6.4), 3.98(1H, dd, J=11.2, 4.2), 4.53(1H, d, J=12.7), 5.05(1H, t, J=10.8), 5.37(1H, dd, J=10.4, 4.2), 7.12(1H, t, J=5.9), about 7.6(2H, bs)

IR : 3300, 1720, 1640, 1557, 1514, 1452, 1325, 1110, 1060 $cm^{-1}$

TLC : Rf = 0.26 (chloroform / methanol, 9 : 1 V/V)

MS (EI): m/z 394 ($M^+$/2-HNCO)

MS (SIMS): m/z 879 [$(M+5)^+$], $C_{38}H_{50}N_8O_{12}S_2$ = 874

Example 14

7-N,7'-N'-[dithiobis(3,6-dioxaoctamethylene)] dimitomycin C (Compound 13)

At first, 175 mg of mitomycin A was dissolved in 10 ml of methanol, and 348 µl of triethylamine and 1.0 ml of a methanol solution of 8,8'-dithiobis-3,6-dioxaoctylamine dihydrochloride (100.4 mg/ml) (see Example 34 for the process for preparing this compound) was added thereto. The mixture was stirred at room temperature for 22.5 hours, and, after further addition of 0.5 ml of said amine dihydrochloride solution thereto, further stirred for 6 hours. Thereafter, operation was carried out in the same manner as in Example 13 [column chromatography was carried out with 50 g of silica gel and chloroform / acetone / methanol (8 : 1 : 1 V/V), whereby 203 mg of Compound 13 was obtained as dark bluish violet powder (yield: 85%)

M.P.: 69 - 75°C

$^1$H-NMR (400 MHz): δ about 2.1(1H, bs), 2.11(3H, s), 2.74(1H, bs), 3.06(2H, t, J=6.4), 3.14(1H, bs), 3.22(3H, s), 3.59(1H, bd, J=12.7), 3.64(2H, t, J=5.4), 3.65(4H, s), 3.74(2H, q, J=5.6), 3.81 (2H, t, J=6.4), 3.98(1H, dd, J=11.2, 4.2), 4.53 (1H, d, J=12.7), 5.06(1H, t, J=10.8), 5.37(1H, dd, J=10.4, 4.2), 7.11(1H, t, J=5.8), about 7.6 (2H, bs)

IR : 3294, 2874, 1719, 1638, 1559, 1509, 1450, 1324, 1112, 1062 $cm^{-1}$

TLC : Rf = 0.42 (chloroform / methanol, 9 : 1 V/V)

MS (EI): m/z 438 ($M^+$/2-HNCO)

MS (SIMS): m/z 963 [$(M+1)^+$], 964 [$(M+2)^+$], 965 [$(M+3)^+$], $C_{42}H_{58}N_8O_{14}S_2$ = 962

Example 15

7-N-[3-(2-pyridyldithio)propyl] mitomycin C (Compound 14)

At first, 1.018 g of mitomycin A and 1.63 ml of triethylamine were dissolved in 15 ml of methanol, and a solution of 797 mg of crude 3-(2-pyridyldithio) propylamine

dihydrochloride (see Reference Example 1) in 10 ml of methanol was dropwise added thereto. The mixture was stirred with ice cooling, and 2.5 hours thereafter 52 mg of mitomycin A was added thereto. The mixtures was left standing in a cold room overnight. Then, the reaction solution was poured into an aqueous saturated sodium bicarbonate solution, and extracted with ethyl acetate (100 ml x 3). The extract was washed with water and then with an aqueous saturated sodium chloride solution, and dried over anhydrous sodium sulfate. After removal of the drying agent therefrom by filtration, the solvent was removed therefrom by distillation under reduced pressure. The residue was dissolved in a small amount of chloroform, and the solution was subjected to silica gel column chromatography (150 g, chloroform : methanol = 96 : 4) and blue fractions showing Rf 0.35 by silica gel thin layer chromatography (chloroform : methanol = 9 : 1) were collected. By removing the solvent therefrom by distillation under reduced pressure and pulverization from n-hexane-chloroform, 524 mg of compound 14 was obtained as dark bluish violet powder (yield: 34.7%).

Example 16 to 19

Compounds 15 to 18 were prepared in the same manner as in Example 15. The raw materials, yields, M.P. and MS of compounds 14 to 18 are shown in Table 5, and IR and NMR data are shown in Table 6.

**Table 5**

Preparation and physical properties of typical compounds (I-1-1)

| Compound | Starting material | | Yield (%) | M.P. (°C) | M S m/z | Molecular formula, Molecular weight |
|---|---|---|---|---|---|---|
| | material mitomycin | amine compound (III) | | | | |
| 14 | Mitomycin A | $\ell$ = 3 | 34.7 | 75 | 520($M^+$ + 3) | $C_{23}H_{27}N_5O_5S_2$, 517 |
| 15 | Mitomycin B | $\ell$ = 3 | 30.0 | 74 - 82 | 520($M^+$ + 3) | $C_{23}H_{27}N_5O_5S_2$, 517 |
| 16 | Mitomycin A | $\ell$ = 4 | 40.0 | 104 | 533($M^+$ + 2) | $C_{24}H_{29}N_5O_5S_2$, 531 |
| 17 | Mitomycin A | $\ell$ = 5 | 58.0 | 135 - 135.5 | 548($M^+$ + 3) | $C_{25}H_{31}N_5O_5S_2$, 545 |
| 18 | Mitomycin A | $\ell$ = 6 | 42.0 | 61 - 72 | 562($M^+$ + 3) | $C_{26}H_{33}N_5O_5S_2$, 559 |

Table 6 IR and NMR data of typical compounds (I-1-1)

| Compound | IR ($cm^{-1}$) | N M R (δ) ($CDCl_3$) |
|---|---|---|
| 14 | 3280, 2930, 1715, 1628, 1550, 1506, 1440, 1323, 1053 | (100MHz) 2.00(3H, s), 2.02(2H, quint, J=6.8), ∿2.8(1H, m), 2,86(2H, t, J=6.8), ∿2.9(1H, m), 3.20(3H, s), 3.51(1H, dd, J=12.9, 2.0), 3.58(1H, dd, J=11.5, 4.6), 3.67(2H, q, J=6.6), 4.28(1H, d, J=12.9), 4.46(1H, t, J=10.9), 4.69(1H, dd, J=10.7, 4.6), 4.91(2H, bs), 6.30(1H, t, J=5.6), ∿7.1(1H, m), 7.65(2H, m), 8.48(1H, m) |
| 15 | 3430, 1708, 1627, 1544, 1509, 1443, 1411, 1330, 1110, 1052 | (400MHz) 1.97(3H, s), 2.01(2H, m), 2.24(1H, d, J=4.7), 2.27 (3H, s), 2.27(1H, dd, J=4.7, 1.9), 2.86(2H, m), 3.51(1H, dd, J=13.0, 1.9), 3.66(2H, q, J=6.6), 3.69(1H, m), 4.16(1H, d, J=13.0), 4.43(1H, bs), 4.70(2H, m), 4.79(2H, bs), 6.28 (1H, t, J=6.2), 7.10(1H, m), 7.64(2H, m), 8.46(1H, m) |
| 16 | 3290, 1720, 1636, 1558, 1514, 1446, 1326, 1060 | (100MHz) 1.76(4H, m), 1.99(3H, s), ∿2.8(1H), 2.82(2H, t, J=5.1), ∿2.9(1H), 3.21(3H, s), 3.51(1H, dd, J=12.9, 1.7), 3.53(2H, q, J=8.1), 3.59(1H, dd, J=11.5, 4.9), 4.30(1H, d, J=12.9), 4.49(1H, t, J=10.7), 4.71(1H, dd, J=10.7, 4.9), 4.74(2H, bs), 6.29(1H, t, J=8.1), 7.09(1H, m), 7.66(2H, m), 8.47(1H, m) |

| Compound | IR ($cm^{-1}$) | NMR (δ) ($CDCl_3$) |
|---|---|---|
| 17 | 3300, 2940, 1721, 1634, 1556, 1512, 1445, 1418, 1324, 1060 | (100MHz) 1.4-1.8(6H, m), 2.00(3H, s), ∿2.8(1H), 2.81(2H, t, J=6.8), ∿2.9(1H), 3.21(3H, s), ∿3.5(1H), ∿3.5(2H), 3.59(1H, dd, J=11.5, 4.6), 4.30(1H, d, J=13.2), 4.49(1H, t, J=11.0), 4.71(1H, dd, J=10.7, 4.6), 4.74(2H, bs), 6.31(1H, t, J=6), 7.09(1H, m), 7.68(2H, m), 8.47(1H, m) |
| 18 | 3300, 2930, 1720, 1634, 1556, 1510, 1444, 1416, 1328, 1060 | (100MHz) 1.3-1.8(8H, m), 2.01(3H, s), ∿2.8(1H), 2.80(2H, t, J=6.8), ∿2.9(1H), 3.21(3H, s), ∿3.5(1H), 3.51(2H, q, J=6.6), 3.59(1H, dd, J=11.5, 4.9), 4.30(1H, d, J=12.9), 4.49(1H, t, J=11.0), 4.71(1H, dd, J=10.5, 4.9), 4.74(2H, bs), 6.32(1H, t, J=5.7), 7.08(1H, m), 7.66(2H, m), 8.46(1H, m) |

Example 20

7-N-[3-[(4-acetamidophenyl)dithio]propyl] mitomycin C (Compound 19)

At first, 149 mg of compound 14 was dissolved in 3 ml of methanol, and then 48 mg of 4-acetamidothiophenol was added thereto with stirring with ice cooling. The mixture was stirred for 20 minutes. After removal of the solvent therefrom by distillation under reduced pressure, the residue was dissolved in a small amount of chloroform, and the solution was subjected to silica gel column chromatography (40 g, chloroform : methanol = 93 : 7), and blue fractions of Rf 0.46 by silica gel thin layer chromatography (chloroform : methanol = 9 : 1) were collected. After removal of the solvent by distillation under reduced pressure, 150 mg of compound 19 was obtained as dark greenish blue powder by pulverization from n-hexane-chloroform (yield: 99%).

Example 21

7-N-[3-[(2-acetamidoethyl)dithio]propyl] mitomycin D (Compound 22)

At first, 10.3 mg of compound 15 was dissolved in 1 ml of methanol, and 4.2 μl of triethylamine was added thereto. Then, 3.1 mg of 2-acetylthioethylamine hydrochloride was added thereto with stirring at room temperature, and the mixture was stirred for one hour. The reaction solution was diluted with ethyl acetate and washed with an aqueous sodium bicarbonate solution. The ethyl acetate layer was washed with water, and then dried over anhydrous sodium sulfate. After removal of the drying agent by filtration, the solvent was removed therefrom by distillation under reduced pressure, and the residue was dissolved in a small amount of chloroform and the solution was subjected to silica gel thin layer chromatography (chloroform : methanol = 9 : 1), and bluish green fractions of Rf 0.23 were scraped and eluted with the same solvent. The eluate was concentrated to dryness under reduced

pressure and pulverized from n-hexane-chloroform, whereby 7.0 mg of compound 22 was obtained as dark green powder (yield: 66%).

Example 22

7-N-[4-(2,3-dihydroxypropyldithio)butyl] mitomycin C (Compound 26)

With 91 mg of compound 16 and 36 μl of 3-mercapto-1,2-propanediol, reaction was carried out in the same manner as in Example 20, and then the reaction solution was subjected to silica gel column chromatography (25 g, chloroform : methanol = 9 : 1) and blue fractions showing Rf 0.26 by silica gel thin layer chromatography (the same solvents) were collected. After removal of the solvent therefrom by concentration under reduced pressure, 79 mg of compound 26 was obtained as dark blue powder by pulverization from n-hexane-chloroform-acetone (yield: 87%)

Example 23

7-N-[5-[(glycino-L-cystein-S-yl)thio]pentyl] mitomycin C (Compound 28)

At first, 301 mg of compound 17 was dissolved in 5 ml of methanol, and the solution was stirred in an ice bath. Then, 95 mg of L-cysteinylglycin was added thereto and the mixture was stirred for 15 minutes. Then, 40 ml of water was added thereto, and the mixture was subjected to adsorption in a column packed with 50 ml of Mitsubishi Diaion HP20 as a carrier. After washing of the column with water, blue fractions eluted with water-methanol (4 : 6) were collected, and concentrated under reduced pressure. By freeze-drying the concentrate, 208 mg of compound 28 was obtained as dark bluish violet powder (yield: 64%). Water solubility of compound 28 can be estimated to be 17 mg/ml based on amount of the concentrate (about 12 ml) and the yield after the freeze-drying (208 mg).

Examples 24 to 30

Compounds 20, 21, 23 - 25, 27 and 29 were prepared in the same manner as in one of Examples 20 - 23. Starting materials and reagents, their amounts, appearance, yield, M.P. and MS data of compounds 19 - 29 are given in Table 7, and IR and NMR data are given in Table 8.

It was clarified in the same manner as in example 23 that water solubilities of compounds 21, 27 and 29 are >29 mg/ml, >39 mg/ml and >19 mg/ml, respectively.

**Table 7**

Preparation and physical properties of typical compounds (I-1-2)

| Starting materials and reagent | Product | Appearance | Yield (%) | M.P. (°C) | MS (m/z) | Molecular formula, Molecular weight |
|---|---|---|---|---|---|---|
| Compound 14 (149 mg) 4-acetamido-thiophenol (48 mg) | Compound 19 | Dark greenish blue powder | 99 | 116 - 129 | 576 ($M^+$ + 3) | $C_{26}H_{31}N_5O_6S_2$ 573 |
| Compound 14 (129 mg) 3-mercapto-1,2-propanediol (41 µl) | Compound 20 | Dark blue solid | 66 | 108 - 116 (decomposed) | 517 ($M^+$ + 3) | $C_{21}H_{30}N_4O_7S_2$ 514 |
| Compound 14 (129 mg) L-cysteinylglycin (42 mg) | Compound 21 | Dark blue powder | 64 | 106 - 109 | 586 ($M^+$ + 2) | $C_{23}H_{32}N_6O_8S_2$ 584 |
| Compound 15 (10.3 mg) 2-acetylthioethyl-amine hydro-chloride (3.1 mg) | Compound 22 | Dark green powder | 66 | 72 - 76 | 528 ($M^+$ + 3) | $C_{22}H_{31}N_5O_6S_2$ 525 |
| Compound 15 (20.7 mg) ethanethiol (21 µl) | Compound 23 | Greyish green powder | 74 | 66 - 68 | 471 ($M^+$ + 3) | $C_{20}H_{28}N_4O_5S_2$ 468 |
| Compound 15 (20.7 mg) cyclohexanethiol (14 µl) | Compound 24 | Greyish green powder | 72 | 45 - 46 | 525 ($M^+$ + 3) | $C_{24}H_{34}N_4O_5S_2$ 522 |

| Starting materials and reagent | Product | Appearance | Yield (%) | M.P. (°C) | MS (m/z) | Molecular formula, Molecular weight |
|---|---|---|---|---|---|---|
| Compound 16 (91 mg) 4-acetamido-thiophenol (29 mg) | Compound 25 | Dark greenish blue powder | 92 | 84 - 92 | 590 ($M^+$ + 3) | $C_{27}H_{33}N_5O_6S_2$ 587 |
| Compound 16 (91 mg) 3-mercapto-1,2-propanediol (36 μl) | Compound 26 | Dark blue powder | 87 | 58 - 65.5 | 531 ($M^+$ + 3) | $C_{22}H_{32}N_4O_7S_2$ 528 |
| Compound 16 (115 mg) L-cysteinylglycin (40 mg) | Compound 27 | Dark blue powder | 62 | 102 - 108 | 600 ($M^+$ + 2) | $C_{24}H_{34}N_6O_8S_2$ 598 |
| Compound 17 (301 mg) L-cysteinylglycin (95 mg) | Compound 28 | Dark bluish violet powder | 64 | 110 (decomposed) | 615 ($M^+$ + 3) | $C_{25}H_{36}N_6O_8S_2$ 612 |
| Compound 18 (210 mg) L-cysteinylglycin (65 mg) | Compound 29 | Dark bluish violet powder | 51 | 110 (decomposed) | 628 ($M^+$ + 2) | $C_{26}H_{38}N_6O_8S_2$ 626 |

**Table 8**

IR and NMR data of typical compounds (I-1-2)

| Compound | IR ($cm^{-1}$) | N M R (δ) |
| --- | --- | --- |
| 19 | 3310, 1712, 1633, 1590, 1552, 1511, 1447, 1329, 1060 | (100MHz, $CDCl_3$) 1.97(3H, s), ∿2.0(2H, t), 2.16(3H, s), 2.75 (2H, t, J=7.1), 2.84(1H, m), 2.92(1H, d, J=4.6), 3.21(3H, s), 3.51(1H, dd, J=12.9, 2.0), ∿3.6(1H), 3.60(2H, q, J=6), 4.29(1H, d, J=12.9), 4.43(1H, t, J=10.5), 4.80(1H, dd, J=10.8, 4.4), 4.83(2H, bs), 6.24(1H, t, J=6.4), 7.51(4H, bs), 8.01(1H, bs) |
| 20 | 3420, 3290, 1710, 1632, 1551, 1512, 1448, 1329, 1216, 1058 | (100MHz, $D_2O$) 2.00(3H, s), 2.06(2H, quint, J=6.6), 2.7-3.1 (6H, m), 3.28(3H, s), 3.5-4.0(7H, m), 4.20(1H, d, J=13.2), 4.24(1H, t, J=10.5), 4.60(1H, dd, J=10.8, 4.6) |
| 21 | 3300, 2950, 1710, 1631, 1602, 1551, 1512, 1470, 1453, 1400, 1331, 1063 | (100MHz, $D_2O$) 1.98(3H, s), 2.06(2H, quint, J=6.8), 2.8(2H, t, J=6.8), 3.0-3.2(4H, m), 3.29(3H, s), 3.4-4.1(7H, m), 4.20(1H, d, J=13.4), 4.23(1H, t, J=10.5), 4.59(1H, dd, J=10.8, 4.9) |

| Compound | IR ($cm^{-1}$) | N M R ($\delta$) |
|---|---|---|
| 22 | 3290, 1708, 1629, 1543, 1508, 1448, 1329, 1050 | (400MHz, Py-$d_5$) 1.90(2H, quint, J=7.0), 2.07(3H, s), 2.07 (3H, s), 2.13(3H, s), 2.23(1H, dd, J=4.7, 1.9), 2.47(1H, d, J=4.7), 2.73(2H, t, J=7.1), 3.00(2H, t, J=6.7), 3.55 (2H, q, J=6.9), 3.68(1H, dd, J=12.9, 1.9), 3.76(2H, q, J=6.6), 4.22(1H, dd, J=10.0, 3.4), 4.45(1H, d, J=12.9), 5.22 (1H, t, J=10.3), 5.46(1H, dd, J=10.6, 3.4), 7.03(1H, t, J=6.4), ∿7.5(2H, bs), 8.33(1H, s), ∿8.7(1H) |
| 23 | 3400, 1710, 1630, 1547, 1510, 1449, 1330, 1052 | (400MHz, Py-$d_5$) 1.22(3H, t, J=7.3), 1.94(2H, quint, J=7.1), 2.09(3H, s), 2.13(3H, s), 2.24(1H, dd, J=4.7, 1.9), 2.48 (1H, d, J=4.7), 2.64(2H, q, J=7.3), 2.72(2H, t, J=7.1), 3.58(2H, q, J=6.8), 3.69(1H, dd, J=12.9, 1.9), 4.24(1H, dd, J=10.0, 3.5), 4.46(1H, d, J=12.9), 5.23(1H, t, J=10.3), 5.48(1H, dd, J=10.5, 3.5), 7.07(1H, t, J=6.5), 7.50(2H, bs), 8.36(1H, bs) |
| 24 | 3280, 2930, 1710, 1630, 1547, 1509, 1447, 1330, 1053 | (400MHz, Py-$d_5$) 1.1-1.5(6H, m), 1.66(2H, m), 1.96(2H, quint, J=7.0), ∿2.0(2H, m), 2.10(3H, s), 2.13(3H, s), 2.23 (1H, dd, J=4.8, 1.9), 2.48(1H, d, J=4.8), 2.75(2H, t, J=7.1), 2.76(1H, tt, J=10.8, 3.8), 3.60(2H, q, J=6.8), 3.69 (1H, dd, J=12.9, 1.9), 4.23(1H, dd, J=10.1, 3.5), 4.45 (1H, d, J=12.9), 5.22(1H, t, J=10.3), 5.48(1H, dd, J=10.5, 3.5), 7.08(1H, t, J=6.5), 7.49(2H, bs), 8.36(1H, bs) |

| Compound | IR ($cm^{-1}$) | NMR (δ) |
|---|---|---|
| 25 | 3310, 1714, 1640, 1594, 1558, 1518, 1470, 1452, 1328, 1060 | (100MHz, $CDCl_3$) 1.67(4H, m), 1.95(3H, s), 2.16(3H, s), ~2.7 (1H), 2.76(2H, t, J=6.6), 2.91(1H, d, J=4.2), 3.20(3H, s), 3.51(1H), 3.52(2H, q, J=7.8), 3.59(1H, dd, J=10.3, 4,4), 4.30(1H, d, J=12.9), 4.42(1H, t, J=10.5), 4.77(1H, dd, J=10.7, 4.4), 5.01(2H, bs), 6.26(1H, t, J=5.9), 7.49(4H, bs), 8.11(1H, bs) |
| 26 | 3440, 3290, 1714, 1638, 1556, 1518, 1450, 1328, 1060 | (100MHz, Py-$d_5$) ~1.7(4H, m), 2.11(3H, s), ~2.7(1H), 2.79 (2H, t, J=6.7), 3.14(1H, m), 3.23(3H, s), ~3.3(2H, m), 3.46(2H, q, J=6.6), 3.60(1H, bd, J=12.7), 3.99(1H, dd, J=10.7, 4.4), 4.11(2H, d, J=5.4), ~4.5(1H, m), 4.55(1H, d, J=12.7), ~5.1(1H, t), 5.38(1H, dd, J=10.5, 4.4), 6.95(1H, t, J=6.5), 7.62(2H, bs) |
| 27 | 3300, 2940, 1716 1634, 1600, 1558, 1519, 1474, 1450, 1326, 1060 | (100MHz, $D_2O$) 1.76(4H, m), 1.98(3H, s), 2.7-3.3(6H, m), 3.29(3H, s), 3.4-4.4(9H, m), 4.60(1H, dd, J=10.5, 4.4) |
| 28 | 3290, 2940, 1720, 1636, 1600, 1560, 1520, 1474, 1450, 1330, 1062 | (100MHz, $D_2O$) 1.3-1.8(6H, m), 1.97(3H, s), 2.6-3.4(6H, m), 3.27(3H, s), 3.4-4.4(9H, m), 4.58(1H, dd, J=10.7, 4.6) |

| Compound | IR ($cm^{-1}$) | N M R (δ) |
|---|---|---|
| 29 | 3300, 2940, 1710, 1636, 1600, 1556, 1514, 1450, 1328, 1060 | (100MHz, $D_2O$) 1.2-1.8(8H, m), 1.96(3H, s), 2.6-3.4(6H, m), 3.27(3H, s), 3.4-4.4(9H, m), 4.58(1H, dd, J=10.7, 4.4) |

Example 31

(1) 5-phthalimido-3-oxapentyl bromide (XIa)

At first, 50 g of 1,5-dibromo-3-oxapentane and 22.2 g of potassium phthalimide were added to 200 ml of acetone, and the mixture was refluxed with stirring and heating for 3 days. After cooling, by-produced potassium bromide was removed therefrom by filtration, and the filtrate was concentrated to dryness. Then, 200 ml of chloroform was added to the residue, and the chloroform layer was washed with water and dried over anhydrous sodium sulfate. After removal of the drying agent therefrom by filtration and chloroform therefrom by distillation, unreacted 1,5-dibromo-3-oxapentane was removed therefrom by distillation under reduced pressure (19.9 g of 1,5-dibromo-3-oxapentane was recovered). The distillation residue was subjected to column chromatography using 400 g of silica gel, and developed by n-hexane / chloroform / ethyl acetate (6 : 3 : 1). Fractions containing compound (XIa) were collected, and the solvent was removed therefrom by distillation. Then, by crystallization from a solvent mixture of n-hexane / acetone, 21.4 g of compound (XIa) was obtained as colorless crystals (yield: 60%).

M.P.: 77.0°C

IR (KBr): 2962, 2932, 2870, 1767, 1690, 1467, 1423, 1393, 1313, 1279, 1107 $cm^{-1}$

NMR (90 MHz, $CDCl_3$): δ 3.39(2H, t, J=6), 3.72-3.91 (6H, m), 7.80(4H, m)

MS (EI): m/z 299 and 297 ($M^+$, calculated value for $C_{12}H_{12}{}^{81}BrNO_3$ = 299), 190, 174, 173, 160

TLC: Rf = 0.48 (n-hexane / chloroform / ethyl acetate, 5 : 3 : 2)

(2) Bis(5-phthalimido-3-oxapentyl) disulfide (IX-1a)

At first, 11.03 g of compound (XIa) and 9.19 g of sodium thiosulfate pentahydrate were added to 80 ml of methanol containing 50% of water, and the mixture was refluxed with stirring and heating for 2 hours. After

disappearance of compound (XIa) has been confirmed by TLC, 60 ml of a methanol solution containing 4.70 g of iodine was added dropwise thereto over 10 minutes. The mixture was further refluxed with heating for one hour. Oily product was separated on the bottom of the reactor vessel, and was recovered by a separating funnel and dissolved in chloroform. Then, the solution was washed successively with water, with an aqueous saturated sodium bicarbonate solution, and with an aqueous saturated sodium chloride solution, and dried over anhydrous sodium sulfate. The reaction product solution freed from the oily product was distilled under reduced pressure to remove methanol therefrom, and then extracted with chloroform. The chloroform layer was subjected to the same operation as above, and dried. After removal of the drying agent therefrom by filtration, both chloroform layers were joined together, concentrated, subjected to chromatography using 150 g of silica gel and eluted with n-hexane / chloroform / ethyl acetate (4 : 3 : 3). Fractions containing the substance whose Rf value by TLC was 0.27 in the same solvent were collected and distilled to remove the solvent therefrom, whereby 8.66 g of compound (IX-1a) was obtained as colorless solid (yield: 93%).

M.P.: 82.0°C

IR (KBr): 2878, 1771, 1704, 1436, 1398, 1125, 1104 $cm^{-1}$

NMR (100 MHz, $CDCl_3$): δ 2.77(2Hx2, t, J=6.6), 3.6-3.9(6Hx2, m), 7.78(4Hx2, m)

MS (EI): m/z 500 ($M^+$, calculated value for $C_{24}H_{24}N_2O_6S_2 = 500$), 309, 283, 250, 218, 192, 190, 175, 174, 160, 147

Example 32

5,5'-dithiobis-3-oxapentylamine (IX-2a)

At first, 2.00 g of compound (IX-1a) and 0.40 g of 80% hydrazine monohydrate were dissolved in 8 ml of ethanol, and the solution was refluxed with heating.

Then, 15 minutes thereafter, 16 ml of ethanol was further added thereto, and one hour thereafter, the mixture was cooled. Then, 1.6 ml of 6N HCl was added thereto, and the precipitated phthalhydrazine was removed therefrom by filtration. Then, 200 ml of water was added to the filtrate, and the neutral and acid substances were removed therefrom by chloroform extraction. The aqueous layer was adjusted to pH 10 with 2N NaOH, and the white turbid aqueous layer was extracted with chloroform. The chloroform layer was washed with an aqueous saturated sodium chloride solution, and dried over anhydrous sodium sulfate. After removal of sodium sulfate by filtration and chloroform by distillation under reduced pressure therefrom, compound (IX-2a) was obtained as a free base. Then, 2 ml of 2N HCl was added thereto, and the mixture was freeze-dried, whereby 967 mg of hydrochloride of compound (IX-2a) was obtained as hygroscopic light yellow solid (yield: 77%). Then, 500 mg of the hydrochloride was subjected to column chromatography using 75 g of silica gel, and developed with a lower layer of chloroform / methanol / concentrated aqueous ammonia (4 : 1 : 1). Fractions showing Rf 0.46 by TLC (the same solvent) were collected, and distilled under reduced pressure to remove the solvent therefrom, whereby 113 mg of colorless oily matter was obtained. Then, 0.94 ml of 1.0N hydrochloric acid was added to the thus obtained oily matter and the mixture was freeze-dried, whereby 159 mg of hydrochloride of compound (IX-2a) for the analytical purpose was obtained.

M.P.: 152 - 157°C

IR (KBr): 3010, 1580, 1560, 1491, 1133, 1102, 1087, 1024 $cm^{-1}$

NMR (100 MHz, $D_2O$): δ 2.96(2Hx2, t, J=6.1), 3.21 (2Hx2, t, J=5.1), 3.78(2Hx2, t, J=5.1), 3.84 (2Hx2, t, J=6.1)

MS (SIMS): m/z 241 ($M^+$+1), $C_8H_{20}N_2O_2S_2$ = 240)

Elemental analysis: Found C: 28.39%, H: 7.21%, N: 8.09%, calculated as $C_8H_{20}N_2O_2S_2 \cdot HCl \cdot 3/2H_2O$ C: 28.23%, H: 7.40%, N:8.23%

Then, 102 mg of the hydrochloride was dissolved in 1 ml of water, and the solution was passed through a column packed with 1 ml of anion exchange resin (Dowex 1 x 4, $OH^-$). The aqueous solution eluted with water was freeze-dried, whereby 72 mg of compound (IX-2a) was obtained as colorless oily matter.

IR (Film method): 3370, 2850, 1590, 1110 $cm^{-1}$

NMR (400 MHz, $D_2O$): δ 2.78(2Hx2, t, J=5.5), 2.96 (2Hx2, t, J=6.0), 3.58(2Hx2, t, J=5.5), 3.82 (2Hx2, t, J=6.0)

MS (EI): m/z 241 ($M^+$+1), 198, 153, 122, 121, 120, 104 (base peak), 93 $C_8H_{20}N_2O_2S_2$ = 240

Elemental analysis: Found C: 39.86%, H: 8.25%, N: 11.11%, calculated as $C_8H_{20}N_2O_2S_2$ C: 39.97%, H: 8.39%, N: 11.65%

Example 33

(1) 8-phthalimido-3,6-dioxaoctyl bromide (XIb)

At first, 60 g of 1,8-dibromo-3,6-dioxaoctane and 22.3 g of potassium phthalimide were added to 250 ml of acetone, and the mixture was refluxed with heating for 3 days. Through the same operation as in Example 31 (1), 28.2 g of 1,8-dibromo-3,6-dioxaoctane was recovered, and the distillation residue was purified by column chromatography using 600 g of silica gel and n-hexane / chloroform / ethyl acetate (5 : 3 : 2 V/V) as an eluting solvent, whereby 30.4 g of 8-phthalimido-3,6-dioxaoctyl bromide (XIb) was obtained as light yellow oily matter (yield: 74%).

IR ($CHCl_3$): 2870, 1771, 1708, 1394, 1120, 1026 $cm^{-1}$

NMR (90 MHz, $CDCl_3$): δ 3.36(2H, t, J=6), 3.65(4H, s), about 3.6-3.9(6H, m), 7.78(4H, m)

MS (EI): m/z 343 and 341 ($M^+$, $C_{14}H_{16}{}^{81}BrNO_4$ = 343), 262, 218, 204, 190, 174, 160

(2) Bis(8-phthalimido-3,6-dioxaoctyl) disulfide (IX-1b)

At first, 13.28 g of compound (XIb) and 9.75 g of sodium thiosulfate pentahydrate were refluxed in 80 ml

of methanol containing 50% of water with heating, and, 2 hours thereafter, 0.49 g of sodium thiosulfate pentahydrate was further added thereto. The mixture was further refluxed for 1.5 hours. After removal of methanol by distillation under reduced pressure and unreacted compound (XIb) by chloroform extraction therefrom, 40 ml of methanol was added to the aqueous layer, and 60 ml of methanol solution containing 4.98 g of iodine was added thereto under reflux with heating. The mixture was further refluxed for one hour. Further operation was carried out in the same manner as in Example 31 (3), and the concentrate of reaction product was purified by column chromatography using 350 g of silica gel and chloroform / ethyl acetate (3 : 1 V/V) as an eluting solvent, whereby 10.46 g of compound (IX-1b) was obtained as colorless oily matter (yield: 92%).

IR ($CHCl_3$): 2870, 1771, 1710, 1393, 1090, 1024 $cm^{-1}$

MS (EI): m/z 588 ($M^+$, calculated value for $C_{28}H_{32}N_2O_8S_2$ = 588), 587, 353, 327, 293, 262, 236, 218, 192, 174)

TLC: Rf = 0.41 (chloroform / ethyl acetate, 7 : 3 V/V)

NMR (90 MHz, $CDCl_3$): δ 2.78(2Hx2, t, J=6), 3.59-3.9 (10Hx2, m), 7.74(4Hx2, m)

Example 34

8,8'-dithiobis-3,6-dioxaoctylamine (IX-2b)

At first, 2.36 g of compound (IX-1b) obtained in Example 33 (2) and 0.40 g of 80% hydrazine monohydrate were dissolved in 24 ml of methanol, and the solution was refluxed with heating for 3 hours. Further, operation was carried out in the same manner as in Examples 32, whereby 1.27 g of dihydrochloride of compound (IX-2b) was obtained as light yellow oily matter (yield: 79%).

Then, 440 mg of the crude hydrochloride was subjected to the same operation as in Example 32, whereby 234 mg of dihydrochloride of compound (IX-2b) was obtained.

TLC: Rf = 0.45 (chloroform / methanol / concentrated aqueous ammonia (lower layer of 4 : 1 : 1)

IR (Film method): 3420, 2900, 1605, 1500, 1100 $cm^{-1}$

NMR (100 MHz, $D_2O$): δ 2.95(2Hx2, t, J=6.1), 3.09(2Hx2, t, J=5.1), 3.71(4Hx2, s), 3.71(2Hx2, t, J=5.1), 3.83(2Hx2, t, J=6.1)

MS (SIMS): m/z 329 ($M^+$+1, $C_{12}H_{28}N_2O_4S_2$ = 328)

Elemental analysis: Found C: 34.42%, H: 7.91%, N: 6.44%, calculated as $C_{12}H_{28}N_2O_4S_2 \cdot 2HCl \cdot H_2O$ C: 34.36%, H: 7.69%, N: 6.68%

Then, 105 mg of the dihydrochloride for analytical purpose was subjected to the same operation as in Example 32 to remove hydrochloric acid therefrom, whereby 82 mg of compound (IX-2b) was obtained as oily matter.

IR (Film method): 3370, 2850, 1590, 1108 $cm^{-1}$

NMR (400 MHz, $D_2O$): δ 2.79(2Hx2, t, J=5.5), 2.97 (2Hx2, t, J=6.0), 3.58(2Hx2, t, J=5.5), 3.71 (4Hx2, m), 3.84(2Hx2, t, J=6.0)

Elemental analysis: Found C: 43.91%, H: 8.53%, N: 8.27%, calculated as $C_{12}H_{28}N_2O_4S_2$ C: 43.88%, H: 8.59%, N: 8.53%

Reference Example 1

Preparation of 3-(2-pyridyldithio) propylamine dihydrochloride

At first, 10.1 g of 3,3'-dithiodipropylamine dihydrochloride was dissolved in 40 ml of water, and then 12.0 ml of 30% aqueous hydrogen peroxide was added thereto. The mixture was left standing at room temperature for 3 days. The solvent was removed therefrom under reduced pressure, and 2.50 g of S-(3'-aminopropyl) 3-aminopropanethiosulfonate dihydrochloride was obtained by crystallization from water-ethanol. Crude S-(3'-aminopropyl) 3-aminopropanethiosulfonate dihydrochloride obtained by freeze drying the said reaction solution as such may be used in the following reaction.

In this reference example, 3.25 g of crude S-(3'-aminopropyl) 3-aminopropanethiosulfonate dihydrochloride was dissolved in 16.8 ml of water and 9.5 ml of ethanol,

and 1.14 ml of concentrated hydrochloric acid was added thereto. Then, 1.27 g of 2-mercaptopyridine was added thereto, and the mixture was stirred at room temperature for 3 days. After removal of ethanol therefrom by distillation under reduced pressure, 50 ml of water was added thereto and extracted with chloroform (50 ml x 3). Then, 3.2 g of potassium hydroxide was added to the aqueous solution with ice cooling, and the mixture was extracted with chloroform (50 ml x 3). The chloroform layer immediately collected was back-extracted with concentrated hydrochloric acid (30 ml x 3), and the hydrochloric acid solution was concentrated to dryness under reduced pressure, and a small amount of water was added to the residue. By freeze drying, 1.67 g of light yellow, crude 3-(2-pyridyldithio) propylamine dihydrochloride was obtained.

Likewise, 4-(2-pyridyldithio) butylamine dihydrochloride, 5-(2-pyridyldithio) pentylamine dihydrochloride, and 6-(2-pyridyldithio) hexylamine dihydrochloride could be obtained from 4,4'-dithiodibutylamine dihydrochloride, 5,5'-dithiodipentylamine dihydrochloride, and 6,6'-dithiodihexylamine dihydrochloride, respectively, in the same manner as above.

## CLAIM

1. A mitomycin derivative represented by the formula (I):

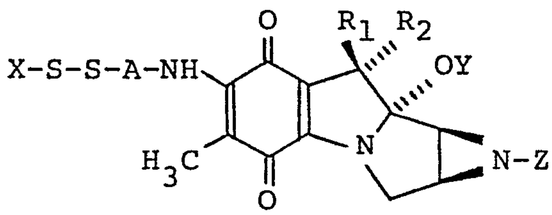

(I)

{wherein, X is $X_1$ or $X_2$, and $X_1$ is an alkyl group having 1 - 7 carbon atoms, a cycloalkyl group having 3 - 7 carbon atoms (the alkyl or cycloalkyl group may be substituted with 1 - 3 of hydroxyl group(s), one amino group or one lower alkanoylamino group), a pyridyl group, an unsubstituted or substituted phenyl group (the substituent is a lower alkyl group, a hydroxyl group, a lower alkoxy group, a nitro group, an amino group, a lower alkylamino group, a lower alkanoylamino group or a halogen atom, and number of the substituent is an integer of 1 - 5), or a residue of an amino acid having a thiol group or a dipeptide or tripeptide containing a residue of the amino acid and the residue being free from the thiol group, where the amino acid, the dipeptide and the tripeptide may be those whose carboxyl groups are protected as lower alkyl ester groups and/or whose amino groups are protected by lower alkanoyl groups, or whose carboxyl groups may form an alkali metal salt, an ammonium salt or an organic amine adduct salt; $X_2$ is a group represented by the formula:

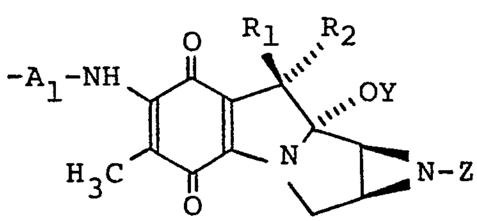

[wherein, $A_1$ is $-(CH_2)_m-$ (wherein, m is an integer of 3 - 12) or $-(CH_2CH_2O)_n-CH_2CH_2-$ (wherein, n is an integer of 1 - 5), and Y, Z, $R_1$ and $R_2$ are as defined in the formula (I)]; when X is $X_1$, A is $-(CH_2)_\ell-$ (wherein, $\ell$ is an integer of 3 - 8) and when X is $X_2$, A is $A_1$; Y and Z are a hydrogen atom or a methyl group; when X is $X_1$, one of $R_1$ and $R_2$ is a carbamoyloxymethyl group, and the other is a hydrogen atom; when X is $X_2$, one of $R_1$ and $R_2$ is a carbamoyloxymethyl group, and the other is hydrogen atom, or $R_1$ and $R_2$ are combined to represent an exo-methylene group $(=CH_2)$}.

2. A mitomycin derivative according to claim 1 wherein X is $X_1$.

3. A mitomycin derivative according to claim 2 wherein $X_1$ is an alkyl group having 1 - 7 carbon atoms, a cycloalkyl group having 3 - 7 carbon atoms (the alkyl or cycloalkyl group may be substituted with 1 - 3 of hydroxyl group(s), one amino group or one lower alkanoyl group), a pyridyl group, or an unsubstituted or substituted phenyl group (the substituent is a lower alkyl group, a hydroxyl group, a lower alkoxy group, a nitro group, an amino group, a lower alkylamino group, a lower alkanoylamino group or a halogen atom, and number of the substituent is an integer of 1 - 5).

4. A mitomycin derivative according to claim 3 wherein $\ell$ is an integer of 4 - 8.

5. A mitomycin derivative according to claim 2 wherein $X_1$ is a residue of an amino acid having a thiol group or a dipeptide or tripeptide containing a residue of the amino acid and the residue being free from the thiol group, where the amino acid, the dipeptide and the tripeptide may be those whose carboxyl groups are protected as lower alkyl ester groups and/or whose amino groups are protected by lower alkanoyl groups, or whose carboxyl

0214303

groups may form an alkali metal salt, an ammonium salt or an organic amine adduct salt.

6. A mitomycin derivative according to claim 5 wherein ℓ is an integer of 4 - 8.

7. A mitomycin derivative according to claim 1 wherein X is $X_2$.

8. A mitomycin derivative according to claim 7 wherein $X_2$ is a group represented by the formula:

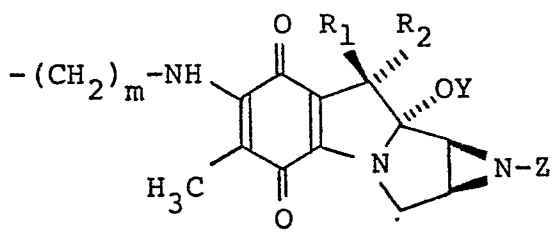

(wherein, m, $R_1$, $R_2$, Y and Z are as defined in claim 1).

9. A mitomycin derivative according to claim 8 wherein m is an integer of 3 - 8.

10. A mitomycin derivative according to claim 7 wherein $X_2$ is a group represented by the formula:

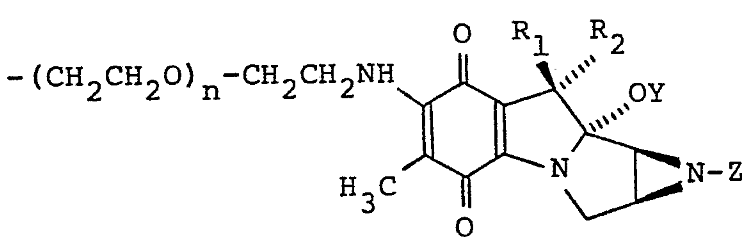

(wherein, n, $R_1$, $R_2$, Y and Z are as defined in claim 1).

11. 7-N,7'-N'-(dithioditrimethylene) dimitomycin C.

12. 7-N,7'-N'-(dithioditetramethylene) dimitomycin C.

13. 7-N,7'-N'-(dithiodipentamethylene) dimitomycin C.

14. 7-N,7'-N'-(dithiohexamethylene) dimitomycin C.

15. 7-N-[4-(4-acetamidophenyldithio)butyl] mitomycin C.

16. 7-N-[3-(2,3-dihydroxypropyldithio)propyl] mitomycin C.

17. 7-N-[4-(2,3-dihydroxypropyldithio)butyl] mitomycin C.

18. 7-N-[3-[(glycino-L-cystein-S-yl)thio]propyl] mitomycin C.

19. 7-N-[5-[(glycino-L-cystein-S-yl)thio]pentyl] mitomycin C.

20. 7-N-[6-[(glycino-L-cystein-S-yl)thio]hexyl] mitomycin C.

21. An anti-tumor agent containing a mitomycin derivative represented by the formula (I):

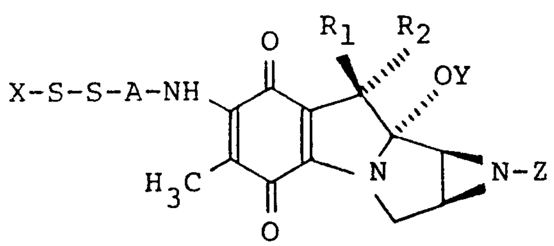

(I)

(wherein, X, A, $R_1$, $R_2$, Y and Z are as defined in claim 1) and at least one of diluents, adjuvants and carriers.

22. A disulfide compound represented by the formula:

$$[-S-(CH_2CH_2O)_n-CH_2CH_2N<^{R_4}_{R_5}]_2$$

(wherein, n is as defined in claim 1, and $R_4$ and $R_5$ are both hydrogen atoms or combined to represent a phthaloyl group) and an acid adduct salt thereof.

0214303

# INTERNATIONAL SEARCH REPORT

International Application No. PCT/JP86/00085

**I. CLASSIFICATION OF SUBJECT MATTER** (if several classification symbols apply, indicate all) [3]

According to International Patent Classification (IPC) or to both National Classification and IPC

Int.Cl[4] C07D487/14, A61K31/40

**II. FIELDS SEARCHED**

Minimum Documentation Searched [4]

| Classification System | Classification Symbols |
|---|---|
| IPC | C07D487/14, A61K31/40 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| | | |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| May 14, 1986 (14. 05. 86) | May 26, 1986 (26. 05. 86) |
| International Searching Authority [1] | Signature of Authorized Officer [20] |
| Japanese Patent Office | |